(19) Europäisches Patentamt
European Patent Office
Office européen des brevets

(11) **EP 2 509 018 B1**

(12) **FASCICULE DE BREVET EUROPEEN**

(45) Date de publication et mention
de la délivrance du brevet:
**11.02.2015  Bulletin 2015/07**

(51) Int Cl.:
**G06F 19/24** *(2011.01)*     **G01N 30/86** *(2006.01)*

(21) Numéro de dépôt: **12162895.2**

(22) Date de dépôt: **02.04.2012**

(54) **Procédé et dispositif d'estimation de paramètres biologiques ou chimiques dans un échantillon, procédé d'aide au diagnostic correspondant**

Verfahren und Vorrichtung zum Schätzen von biologischen oder chemischen Parametern in einer Probe, entsprechendes Diagnose-Hilfsverfahren

Method and device for estimating biological or chemical parameters in a sample, corresponding diagnosis assistance method

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **06.04.2011  FR 1153008**

(43) Date de publication de la demande:
**10.10.2012  Bulletin 2012/41**

(73) Titulaires:
• **Commissariat à l'Énergie Atomique et aux Énergies Alternatives**
**75015 Paris (FR)**
• **UNIVERSITE DE BORDEAUX**
**33000 Bordeaux (FR)**

(72) Inventeurs:
• **Szacherski, Pascal**
**38380 Saint Laurent Du Pont (FR)**
• **Giovannelli, Jean-François**
**75014 Paris (FR)**
• **Grangeat, Pierre**
**38330 Saint Ismier (FR)**

(74) Mandataire: **Bonnet, Michel**
**Cabinet Bonnet**
**93, Rue Réaumur - Boîte 10**
**75002 Paris (FR)**

(56) Documents cités:
**EP-A1- 2 028 486**

• **KANG H D ET AL: "Decomposition of multicomponent mass spectra using Bayesian probability theory", JOURNAL OF MASS SPECTROMETRY, WILEY, CHICHESTER, GB, vol. 37, 1 juillet 2002 (2002-07-01), pages 748-754, XP002476266, ISSN: 1076-5174, DOI: 10.1002/JMS.335 [extrait le 2002-06-12]**
• **SCHWARZ-SELLINGER T ET AL: "Analysis of multicomponent mass spectra applying Bayesian probability theory", JOURNAL OF MASS SPECTROMETRY, WILEY, CHICHESTER, GB, vol. 36, 1 août 2001 (2001-08-01), pages 866-874, XP002476267, ISSN: 1076-5174, DOI: 10.1002/JMS.187 [extrait le 2001-08-07]**
• **MOUSSAOUI SAID ET AL: "Separation of Non-Negative Mixture of Non-Negative Sources Using a Bayesian Approach and MCMC Sampling", IEEE TRANSACTIONS ON SIGNAL PROCESSING, IEEE SERVICE CENTER, NEW YORK, NY, US, vol. 54, no. 11, 1 novembre 2006 (2006-11-01), pages 4133-4145, XP002476268, ISSN: 1053-587X, DOI: 10.1109/TSP.2006.880310**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention concerne un procédé et un dispositif d'estimation de paramètres biologiques ou chimiques dans un échantillon. Elle concerne également un procédé d'aide au diagnostic correspondant.

Une application particulièrement prometteuse d'un tel procédé est par exemple l'analyse d'échantillons biologiques tels que des prélèvements sanguins ou de plasma pour en extraire des paramètres biologiques tels qu'une estimation de concentrations moléculaires en protéines. La connaissance de ces concentrations permet de détecter des anomalies ou maladies. Notamment, il est connu que certaines maladies telles que des cancers, même à un stade peu avancé, peuvent avoir un impact éventuellement décelable sur les concentrations moléculaires de certaines protéines. Plus généralement, l'analyse d'échantillons pour l'extraction de paramètres pertinents permettant une aide au diagnostic d'un état (santé, pollution, ...) pouvant être associé à ces échantillons est un domaine d'application prometteur d'un procédé selon l'invention.

Parmi les applications concrètes envisageables, on peut noter les suivantes : l'analyse biologique d'échantillons par détection de protéines ; la caractérisation de bactéries par spectrométrie de masse ; la caractérisation de l'état de pollution d'un échantillon chimique (par exemple, le dosage d'un gaz dans un environnement ou le dosage d'un métal lourd dans un échantillon liquide). Les paramètres pertinents extraits peuvent comporter des concentrations de composants tels que des molécules (peptides, protéines, enzymes, anticorps, ...) ou des assemblages moléculaires. Par assemblage moléculaire on entend par exemple une nanoparticule ou une espèce biologique (bactérie, microorganisme, cellule, ...).

Dans le cas d'une analyse biologique par détection de protéines, toute la difficulté est de parvenir à une estimation la plus précise possible dans un environnement bruité où les protéines d'intérêt sont parfois présentes dans l'échantillon en très petite quantité.

En général, l'échantillon passe par une chaîne de traitement comportant par exemple une colonne de chromatographie ou un spectromètre de masse, ou les deux. Cette chaîne de traitement est conçue pour la fourniture d'un signal représentatif de concentrations moléculaires des composants dans l'échantillon en fonction d'un temps de rétention dans la colonne de chromatographie ou d'un rapport masse/charge dans le spectromètre de masse, ou des deux.

Eventuellement, la chaîne de traitement peut comporter, en amont de la colonne de chromatographie, une centrifugeuse et/ou une colonne de capture par affinité, de manière à purifier l'échantillon. Elle peut en outre comporter, également en amont de la colonne de chromatographie et lorsque les composants à étudier sont des protéines, une colonne de digestion sectionnant les protéines en peptides plus petits, donc mieux adaptés à la gamme de mesure du spectromètre de masse. Enfin, lorsque la chaîne de traitement comporte à la fois la colonne de chromatographie, devant être traversée par un échantillon en phase liquide, et le spectromètre de masse, imposant que l'échantillon soit en phase gazeuse nébulisée, elle doit en outre comporter un électrospray (ou équivalent) apte à procéder au changement de phase nécessaire, en l'occurrence par nébulisation du mélange fourni en sortie de la colonne de chromatographie.

Ainsi, lorsque la chaîne de traitement comporte la colonne de chromatographie et le spectromètre de masse, en adaptant judicieusement la période d'échantillonnage temporel des mesures du spectromètre de masse à un multiple de la période d'échantillonnage temporel des mesures de la colonne de chromatographie, il est possible de fournir un signal bidimensionnel dont l'amplitude positive varie en fonction du temps de rétention dans la colonne de chromatographie dans une dimension et du rapport masse/charge identifié par le spectromètre de masse dans l'autre dimension. Ce signal bidimensionnel présente une multitude de pics révélateurs de concentrations de composants plus ou moins noyés dans du bruit et plus ou moins superposés les uns aux autres.

Un procédé connu d'estimation des concentrations des composants consiste à mesurer la hauteur des pics ou leur intégrale (surface, volume) au delà d'un certain niveau et d'en déduire la concentration d'un composant correspondant. Un autre procédé dit « d'analyse spectrale » consiste à comparer le signal bidimensionnel dans son ensemble à une bibliothèque de modèles répertoriés. Mais ces procédés souffrent généralement d'un manque de précision ou de fiabilité, notamment lorsque les pics sont peu marqués ou rendus moins visibles à cause de bruit ou de pics très voisins qui se superposent.

Un autre procédé connu consiste à exprimer analytiquement la chaîne de traitement et obtenir ainsi un modèle direct du signal fourni en sortie, pour ensuite effectuer une estimation des paramètres biologiques ou chimiques par inversion de ce modèle à l'aide des valeurs effectivement observées du signal et d'une inférence bayésienne. Un tel procédé est notamment décrit dans la demande de brevet européen publiée sous le numéro EP 2 028 486. Il comporte les étapes suivantes :

- faire passer l'échantillon par une chaîne de traitement,
- obtenir ainsi un signal représentatif de concentrations de composants de l'échantillon en fonction d'au moins une variable de la chaîne de traitement, et
- estimer les concentrations à l'aide d'un dispositif de traitement de signal par inférence bayésienne, sur la base d'une modélisation analytique directe dudit signal en fonction de paramètres biologiques de l'échantillon, parmi lesquels se trouve un vecteur représentatif des concentrations desdits composants, et de paramètres techniques de la chaîne de traitement.

La modélisation analytique proposée dans ce document rend le signal de chromato-spectrographie observé dépendant notamment des paramètres biologiques et techniques suivants : un vecteur représentatif des concentrations de P protéines, un vecteur représentatif des concentrations de 1 peptides issus d'une digestion des P protéines, un paramètre général de gain de la chaîne de traitement, un paramètre de bruit, un paramètre de temps de rétention dans la colonne de chromatographie. Les valeurs de certains de ces paramètres sont variables ou inconnues d'une chromato-spectrographie à l'autre. Ces paramètres sont alors modélisés par des lois de probabilités indépendantes les unes des autres (vecteur des concentrations de protéines, paramètre de gain, paramètre de bruit et paramètre de temps de rétention), tandis que d'autres sont obtenus de façon déterministe, par apprentissage (tel le vecteur des concentrations moléculaires de peptides déterminé à partir du vecteur des concentrations de protéines et d'une matrice de digestion invariable) ou éventuellement par calibrage de la chaîne de traitement.

Il se trouve cependant que le modèle choisi dans EP 2 028 486 présente des rigidités ayant un impact sur la précision et la fiabilité de l'estimation finale. Il peut ainsi être souhaité de prévoir un procédé d'estimation de paramètres biologiques ou chimiques qui permette de s'affranchir d'au moins une partie des problèmes et contraintes précités et d'améliorer les procédés existants.

Il est donc proposé un procédé d'estimation de paramètres biologiques ou chimiques dans un échantillon, comportant les étapes suivantes :

- faire passer l'échantillon par une chaîne de traitement,
- obtenir ainsi un signal représentatif des paramètres biologiques ou chimiques en fonction d'au moins une variable de la chaîne de traitement, et
- estimer les paramètres biologiques ou chimiques à l'aide d'un dispositif de traitement de signal par inférence bayésienne, sur la base d'une modélisation analytique directe dudit signal en fonction des paramètres biologiques ou chimiques et de paramètres techniques de la chaîne de traitement,

dans lequel, en outre :

- au moins deux des paramètres biologiques ou chimiques ou techniques en fonction desquels la modélisation analytique directe dudit signal est définie ont une relation de dépendance probabiliste entre eux, et
- le traitement de signal par inférence bayésienne est en outre réalisé sur la base d'une modélisation par loi de probabilité a priori conditionnelle de cette dépendance.

Ainsi, la modélisation peut être affinée, et donc s'approcher de la réalité, en intégrant une hiérarchie de certains paramètres à l'aide de dépendances probabilistes traduites par des lois de probabilités conditionnelles, sachant que ces dépendances probabilistes peuvent être modélisées a priori, par exemple soit par un apprentissage spécifique, soit par un modèle réaliste imposé par l'expérience. Il en résulte finalement une meilleure estimation des paramètres biologiques ou chimiques concernés.

De façon optionnelle, l'étape d'estimation des paramètres biologiques ou chimiques comporte, par approximation de la loi de probabilité a posteriori jointe des paramètres biologiques ou chimiques et des paramètres techniques conditionnellement au signal obtenu à l'aide d'un algorithme d'échantillonnage stochastique :

- une boucle d'échantillonnage d'au moins une partie des paramètres biologiques ou chimiques de l'échantillon et d'au moins une partie des paramètres techniques de la chaîne de traitement, fournissant des valeurs échantillonnées de ces paramètres, et
- une estimation de ladite au moins une partie des paramètres biologiques ou chimiques et techniques calculée à partir des valeurs échantillonnées fournies.

Ainsi, sur la base d'une connaissance de modèles de lois de probabilités a priori, conditionnelles ou non, d'au moins une partie des paramètres biologiques et/ou techniques, il devient possible de traiter simplement le signal fourni par la chaîne de traitement biologique pour en extraire des estimations de ces paramètres.

De façon optionnelle également, l'estimation de ladite au moins une partie des paramètres biologiques ou chimiques et techniques calculée à partir des valeurs échantillonnées fournies comporte :

- un calcul de l'estimateur espérance ou médiane ou maximum a posteriori pour chaque paramètre à valeurs continues,
- un calcul de l'estimateur maximum a posteriori pour chaque paramètre à valeurs discrètes, ou
- un calcul de probabilité d'au moins une partie des paramètres biologiques ou chimiques et techniques.

De façon optionnelle également, les paramètres biologiques ou chimiques comportent un vecteur représentatif de concentrations de composants de l'échantillon, le procédé comportant en outre une phase préalable de calibrage, dit calibrage externe, comportant les étapes suivantes :

- faire passer un échantillon de composants de calibrage externe par la chaîne de traitement, ces composants de calibrage externe étant choisis parmi les composants de l'échantillon et étant de concentration connue,
- obtenir ainsi un signal représentatif des concentrations des composants de calibrage externe en fonction d'au moins une variable de la chaîne de traitement et d'au moins un paramètre constant de valeur inconnue et/ou d'au moins un paramètre statistique stable de la chaîne de traitement,
- appliquer au moins une partie de l'étape d'estimation des paramètres biologiques ou chimiques à l'aide du dispositif de traitement de signal par inférence bayésienne, pour en déduire la valeur de chaque paramètre constant de valeur inconnue et/ou de chaque paramètre statistique stable de la chaîne de traitement,
- enregistrer en mémoire chaque valeur de paramètre constant et/ou chaque valeur de paramètre statistique stable déduite précédemment.

De façon optionnelle également, les paramètres biologiques ou chimiques sont relatifs à des protéines et l'échantillon comporte l'un des éléments de l'ensemble constitué de sang, plasma et urine.

De façon optionnelle également :

- le signal représentatif des paramètres biologiques ou chimiques est exprimé en fonction de concentrations moléculaires d'espèces,
- ces espèces sont issues d'une décomposition d'espèces moléculaires d'intérêt,
- le procédé comporte une estimation d'une quantité de ces espèces obtenue suite à la décomposition des espèces moléculaires d'intérêt.

De façon optionnelle également :

- les espèces comportent des peptides ou des polypeptides,
- les espèces moléculaires d'intérêt comportent des protéines contenant chacune une quantité de ces peptides ou polypeptides,
- on définit un rendement de digestion des protéines sous la forme d'une matrice de coefficients $\alpha_{ip}$, où $\alpha_{ip}$ désigne le rendement de digestion de la p-ième protéine par rapport au i-ième peptide ou polypeptide, de sorte que les concentrations moléculaires de peptides ou polypeptides soient liées à un vecteur représentatif de concentrations des protéines via une matrice de digestion et ce rendement de digestion,
- le procédé comporte une estimation de ce rendement de digestion.

De façon optionnelle également :

- les espèces comportent des peptides ou des polypeptides,
- les espèces moléculaires d'intérêt comportent des protéines contenant une quantité de ces peptides ou polypeptides,
- on définit un gain globale $\xi$ de la chaîne de traitement de manière à modéliser le signal Y représentatif des paramètres biologiques ou chimiques par la relation $Y = \xi K$, où K est un vecteur représentatif de concentrations des peptides ou polypeptides,
- le procédé comporte une estimation de ce gain global.

Il est également proposé un procédé d'aide au diagnostic comportant les étapes d'un procédé d'estimation de paramètres biologiques ou chimiques tel que défini précédemment, dans lequel les paramètres biologiques ou chimiques de l'échantillon comportent un paramètre d'état biologique ou chimique à valeurs discrètes, chaque valeur discrète possible de ce paramètre étant associée à un état possible de l'échantillon, et un vecteur représentatif de concentrations de composants de l'échantillon, et dans lequel le vecteur représentatif des concentrations et le paramètre d'état biologique ou chimique ayant une relation de dépendance probabiliste entre eux, le traitement de signal par inférence bayésienne est en outre réalisé sur la base d'une modélisation par loi de probabilité a priori du vecteur représentatif des concentrations conditionnellement aux valeurs possibles du paramètre d'état biologique ou chimique.

De façon optionnelle, un procédé d'aide au diagnostic selon l'invention comporte une phase préalable d'apprentissage comportant les étapes suivantes :

- faire passer successivement une pluralité d'échantillons de référence par la chaîne de traitement, la valeur du paramètre d'état biologique ou chimique de chaque échantillon de référence étant connue,

- obtenir ainsi un signal représentatif des concentrations des composants pour chaque échantillon de référence en fonction d'au moins une variable de la chaîne de traitement,
- appliquer au moins une partie de l'étape d'estimation des paramètres biologiques ou chimiques à l'aide du dispositif de traitement de signal par inférence bayésienne, pour en déduire des valeurs des concentrations des composants pour chaque échantillon de référence,
- en déduire des paramètres de loi de probabilité a priori du vecteur représentatif des concentrations conditionnellement aux valeurs possibles du paramètre d'état biologique ou chimique, et
- enregistrer en mémoire ces paramètres de loi de probabilité.

De façon optionnelle également, un procédé d'aide au diagnostic selon l'invention comporte une phase préalable de sélection desdits composants parmi un ensemble de composants candidats, cette phase préalable de sélection comportant les étapes suivantes :

- faire passer successivement une pluralité d'échantillons de référence par la chaîne de traitement, la valeur du paramètre d'état biologique ou chimique de chaque échantillon de référence étant connue,
- obtenir ainsi un signal représentatif des concentrations des composants candidats pour chaque échantillon de référence en fonction d'au moins une variable de la chaîne de traitement,
- appliquer au moins une partie de l'étape d'estimation des paramètres biologiques ou chimiques à l'aide du dispositif de traitement de signal par inférence bayésienne, pour en déduire des valeurs représentatives des concentrations des composants candidats pour chaque échantillon de référence,
- en déduire des paramètres de distributions du vecteur représentatif des concentrations des composants candidats pour chaque valeur discrète du paramètre d'état biologique ou chimique,
- sélectionner, parmi les composants candidats, ceux dont les distributions sont les plus dissemblables les unes des autres en fonction des valeurs du paramètre d'état biologique ou chimique.

Enfin, il est également proposé un dispositif d'estimation de paramètres biologiques ou chimiques dans un échantillon, comportant :

- une chaîne de traitement de l'échantillon conçue pour la fourniture d'un signal représentatif des paramètres biologiques ou chimiques en fonction d'au moins une variable de la chaîne de traitement,
- un dispositif de traitement de signal conçu pour appliquer, en combinaison avec la chaîne de traitement, un procédé d'estimation de paramètres biologiques ou chimiques ou d'aide au diagnostic tel que défini précédemment.

De façon optionnelle, la chaîne de traitement comporte une colonne de chromatographie et/ou un spectromètre de masse et est conçue pour la fourniture d'un signal représentatif des concentrations de composants de l'échantillon en fonction d'un temps de rétention dans la colonne de chromatographie et/ou d'un rapport masse/charge dans le spectromètre de masse.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif d'estimation de paramètres biologique ou chimiques et d'aide au diagnostic selon un mode de réalisation de l'invention,
- la figure 2 illustre une modélisation analytique hiérarchique d'une chaîne de traitement du dispositif de la figure 1, selon un mode de réalisation de l'invention, et
- la figure 3 illustre les étapes successives d'un procédé d'estimation de paramètres biologique ou chimiques et d'aide au diagnostic selon un mode de réalisation de l'invention.

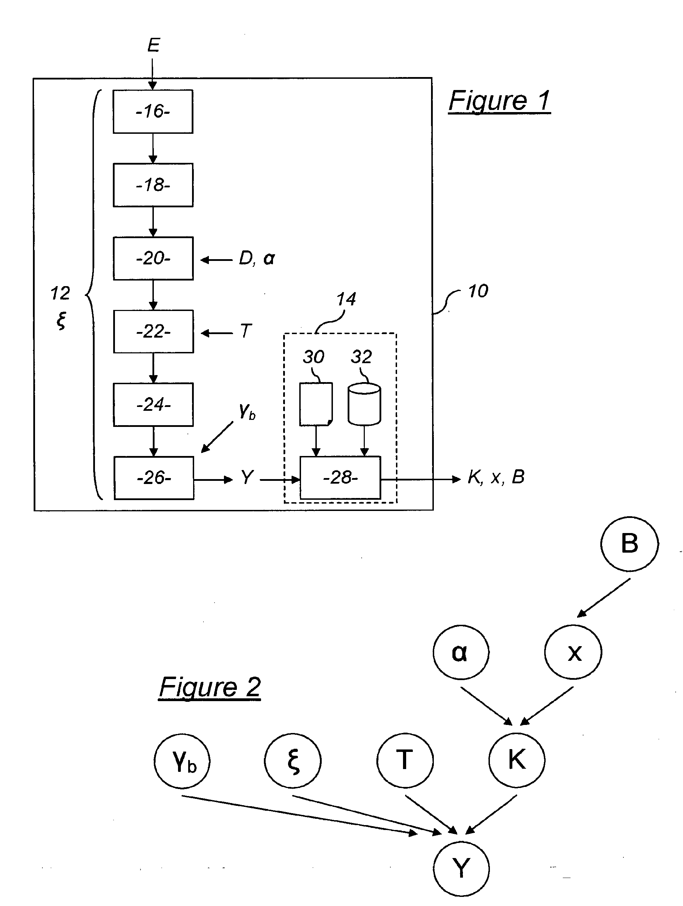

Le dispositif 10 d'estimation de paramètres biologiques ou chimiques dans un échantillon E, représenté schématiquement sur la figure 1, comporte une chaîne 12 de traitement de l'échantillon E conçue pour la fourniture d'un signal Y représentatif de ces paramètres biologiques ou chimiques en fonction d'au moins une variable de la chaîne de traitement 12. Il comporte en outre un dispositif 14 de traitement de signal conçu pour appliquer, en combinaison avec la chaîne de traitement 12, un procédé d'estimation des paramètres biologiques ou chimiques et d'aide au diagnostic en fonction de ces paramètres.

Dans l'exemple qui va être détaillé par la suite mais qui ne doit pas être considéré comme limitatif, les paramètres estimés sont des paramètres biologiques, parmi lesquels des concentrations de composants biologiques de l'échantillon E considéré alors comme un échantillon biologique, et la chaîne de traitement 12 est une chaîne de traitement biologique. Plus précisément, les composants sont des protéines d'intérêt, par exemple sélectionnées en fonction de leur pertinence pour caractériser une anomalie, affection ou maladie, et l'échantillon E est un échantillon de sang, de plasma ou d'urine.

On parlera alors de concentrations moléculaires de protéines pour désigner les concentrations de ces composants particuliers.

Dans la chaîne de traitement biologique 12, l'échantillon E passe tout d'abord par une centrifugeuse 16, puis par une colonne de capture par affinité 18, de manière à être purifié.

Il traverse ensuite une colonne de digestion 20 sectionnant ses protéines en peptides plus petits à l'aide d'une enzyme, par exemple la trypsine. La digestion peut être modélisée par une matrice de digestion D, définissant de manière déterministe comment chaque protéine d'intérêt est sectionnée en peptides, et par un coefficient de digestion $\alpha$ caractérisant le rendement de cette digestion. Ce coefficient $\alpha$ peut être qualifié de paramètre incertain dans le sens où il est susceptible de varier de façon aléatoire d'un traitement biologique à l'autre. Il peut donc avantageusement être modélisé selon une loi de probabilité a priori prédéterminée, par exemple une loi uniforme sur un intervalle inclus dans [0,1].

Puis l'échantillon E passe successivement par une colonne de chromatographie liquide 22, un électrospray 24 et un spectromètre de masse 26, pour la fourniture du signal Y qui est alors représentatif des concentrations moléculaires des protéines dans l'échantillon E en fonction d'un temps de rétention dans la colonne de chromatographie 22 et d'un rapport masse/charge dans le spectromètre de masse 26. Ce signal Y peut ainsi être qualifié de chromatospectrogramme. Comme indiqué précédemment, par une bonne adaptation de la période d'échantillonnage temporel des mesures du spectromètre de masse 26 à un multiple de la période d'échantillonnage temporel des mesures de la colonne de chromatographie 22, le signal observé Y est fourni sous la forme d'un signal bidimensionnel dont l'amplitude positive varie en fonction du temps de rétention dans la colonne de chromatographie 22 dans une dimension et du rapport masse/charge dans le spectromètre de masse 26 dans l'autre dimension.

La séparation des peptides dans la colonne de chromatographie 22 se fait en fonction de leur temps de rétention T dans cette colonne. Ce paramètre T est lui aussi un paramètre incertain puisqu'il est susceptible de varier de façon aléatoire d'un traitement biologique à l'autre. Il peut donc avantageusement être modélisé selon une loi de probabilité a priori prédéterminée.

La sortie observable Y du spectromètre de masse 26 peut être considérée comme perturbée par un bruit dont l'inverse variance est définie par un paramètre $\gamma_b$. Ce paramètre de bruit est lui aussi un paramètre incertain susceptible de varier de façon aléatoire d'un traitement biologique à l'autre. Il peut donc avantageusement être modélisé selon une loi de probabilité a priori prédéterminée.

Enfin, d'une façon globale, la chaîne de traitement 12 présente un gain $\xi$ qui, lui aussi, est un paramètre incertain susceptible de varier de façon aléatoire d'un traitement biologique à l'autre. Il peut donc avantageusement être modélisé selon une loi de probabilité a priori prédéterminée.

Le signal bidimensionnel Y est fourni en entrée du dispositif de traitement 14. Plus précisément, le dispositif de traitement 14 comporte un processeur 28 relié à des moyens de stockage comportant notamment au moins une séquence programmée d'instructions 30 et une base de données de modélisation 32.

La base de données 32 comporte les paramètres d'une modélisation analytique directe du signal Y en fonction :

- de paramètres biologiques de l'échantillon E, parmi lesquels se trouvent un vecteur x représentatif des concentrations moléculaires des protéines d'intérêt et un paramètre d'état biologique B à valeurs discrètes, chaque valeur discrète possible de ce paramètre étant associée à un état prédéterminé possible de l'échantillon E,
- des paramètres techniques précités D, $\alpha$, T, $\xi$ et $\gamma_b$ de la chaîne de traitement biologique 12, et
- d'un autre paramètre technique K, vecteur représentatif des concentrations moléculaires des peptides obtenus par digestion des protéines d'intérêt, directement lié au vecteur x via les paramètres D et $\alpha$.

On notera que le paramètre de gain $\xi$ n'est pas connu de façon absolue et sans cette connaissance absolue, il n'est pas possible d'estimer correctement la concentration x des protéines d'intérêt. En pratique, ce problème est résolu par l'insertion de protéines de marquage équivalentes aux protéines d'intérêt (mais de masse différente) dans l'échantillon E avant le passage de ce dernier dans la chaîne de traitement 12. La concentration x* de ces protéines de marquage est connue, de sorte que le gain ainsi que la concentration x peuvent alors être estimés à l'aide de x* et d'une comparaison des pics correspondant aux protéines d'intérêt et aux protéines de marquage dans le signal observé Y.

La modélisation analytique directe du signal Y prend ainsi la forme :

$$Y = \sum_{i=1}^{I} \sum_{j=1}^{J} \sum_{K=1}^{K} \xi_i \pi_{ij} \left( K_i \pi'_{ijk} \, s_{ijk} + K_i^* \pi'^*_{ijk} \, s_{ijk}^* \right) c_i^T (T_i) + b(\gamma_b), \qquad (1)$$

avec o $K_i = \sum_{p=1}^{P} x_p \alpha_{ip} d_{ip}$ , où I est le nombre de peptides, J le nombre de charges et K

le nombre de neutrons supplémentaires que peut avoir un peptide, P le nombre de protéines d'intérêts, $x_p$ la concentration moléculaire en p-ième protéine d'intérêt, $\alpha_{ip}$ le rendement de digestion de la p-ième protéine par rapport au i-ième peptide, $d_{ip}$ le nombre de i-ièmes peptides fournis par la digestion de la p-ième protéine, $\xi_i$ le gain de la chaîne de traitement biologique relatif au i-ième peptide, $\pi_{ij}$ le pourcentage de i-ième peptide ayant j charges, $\pi'_{ijk}$ le pourcentage de i-ième peptide avec j charges ayant k neutrons supplémentaires, $s_{ijk}$ le spectre théorique discrétisé du peptide i portant j charges et k neutrons supplémentaires, $K_i$ la concentration moléculaire en i-ième peptide, $c_i^T(T_i)$ le débit moléculaire du i-ième peptide dans la colonne de chromatographie, $b(\gamma_b)$ le modèle de bruit et où le symbole « * » désigne, le cas échéant, les mêmes paramètres associés aux protéines de marquage. $\alpha_{ip}$ est compris entre 0 et 1. Le vecteur K, comprenant les concentrations $K_i$ de chaque peptide i dans un volume égal au volume initial de l'échantillon de protéines, peut contenir non seulement des peptides isolés, c'est-à-dire bien digérés, mais également des polypeptides, par exemple issus d'une mauvaise digestion, ces derniers étant alors assimilés à un peptide dans le modèle. Dans ce cas, la matrice D définit la quantité de peptides et de polypeptides produits par la digestion d'une protéine et les coefficients $\alpha_{ip}$ représentent un rendement qui peut être variable pour différents peptides i d'une protéine p. Cela entraîne une prise en compte des peptides bien digérés, mais également des polypeptides mal digérés. Tous les peptides sont cependant traités de la même manière, les polypeptides mal digérés étant assimilés à un peptide dans la modélisation.

Sur fourniture du signal effectivement observé Y, la séquence programmée d'instructions 30 est conçue pour résoudre l'inversion de ce modèle analytique dans un cadre bayésien par une estimation a posteriori basée sur des modèles de probabilité, par exemple des modèles a priori, d'au moins une partie des paramètres précités.

La séquence d'instructions 30 et la base de données 32 sont fonctionnellement présentées comme distinctes sur la figure 1, mais en pratique elles peuvent êtres réparties différemment en fichiers de données, codes sources ou librairies informatiques sans que cela ne change quoi que ce soit aux fonctions remplies.

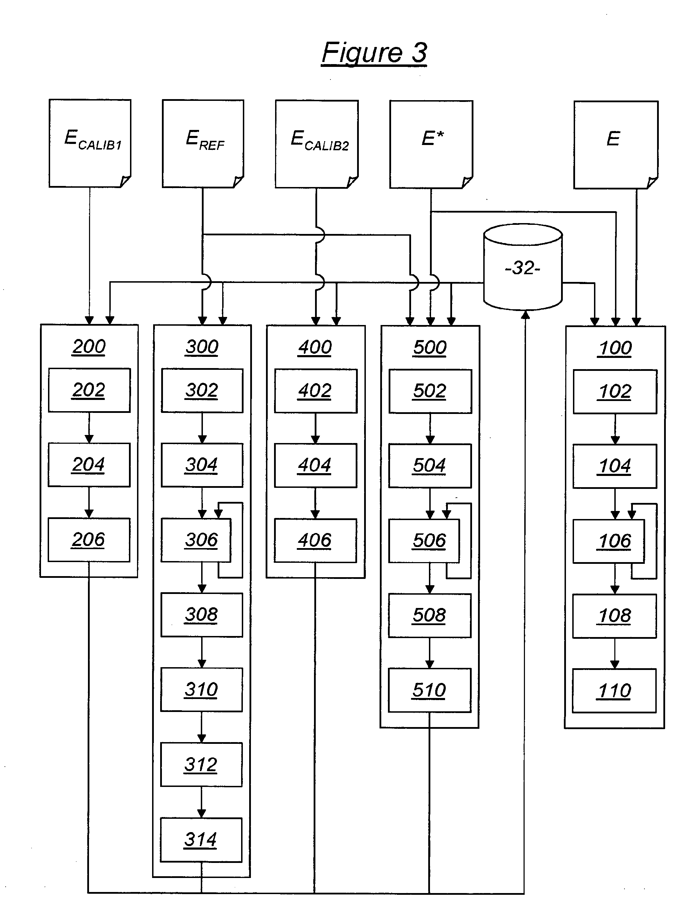

Certains des paramètres précités sont incertains et sont modélisés a priori par des lois de probabilité continues ou discrètes: il s'agit des paramètres techniques $\gamma_b$, $\xi$, T, K, K* et $\alpha$ de la chaîne de traitement biologique 12 et des paramètres biologiques, x et B. Ce sont ces paramètres, parmi lesquels se trouvent le vecteur x (pour une estimation des concentrations moléculaires de protéines) et le paramètre d'état biologique B (pour une aide au diagnostic), qui sont estimés par inversion du modèle direct selon un procédé qui sera détaillé en référence à la figure 3.

Comme illustré sur la figure 2, certains de ces paramètres incertains sont définis comme ayant une relation de dépendance probabiliste entre eux, conduisant à un modèle probabiliste global hiérarchique.

Ainsi, dans notre exemple particulier, le vecteur x représentatif des concentrations moléculaires de protéines d'intérêt est défini comme dépendant du paramètre d'état biologique B. En effet, on conçoit que de façon réaliste, la variable aléatoire x suit une loi de probabilité qui varie en fonction de l'état associé à l'échantillon E.

De même, le vecteur K représentatif des concentrations moléculaires de peptides est dépendant du vecteur x et de la matrice de digestion $\alpha$ comprenant les termes $\alpha_{ip}$, ce qui correspond bien à notre modèle réaliste de digestion à rendement inférieur à 1.

De même, le vecteur K* représentatif des concentrations moléculaires de peptides issus des protéines marquées est dépendant du vecteur constant et connu x* et de la matrice de digestion $\alpha$, ce qui correspond bien à notre modèle réaliste de digestion à rendement inférieur à 1.

Par conséquent, à un premier niveau de hiérarchie du modèle probabiliste, le signal observé Y dépend uniquement des variables aléatoires $\gamma_b$, $\xi$, T, K et K*. A un deuxième niveau de hiérarchie du modèle probabiliste, le vecteur K dépend uniquement des variables aléatoires $\alpha$ et x, et K* de la variable aléatoire $\alpha$ et de la variable fixe x*. Enfin, à un troisième niveau de hiérarchie du modèle probabiliste, le vecteur x dépend de la variable aléatoire à valeurs discrètes B. On remarque en particulier que ce modèle hiérarchique engendre, par la dépendance définie entre K et x (et également K* et x*) via $\alpha$, une hiérarchie entre les paramètres biologiques et les paramètres techniques : il présente ainsi en effet un premier étage technique, dépendant d'un second étage biologique, chacun de ces deux étages pouvant lui-même présenter une hiérarchie interne en fonction du modèle retenu.

Sur la base de ce modèle probabiliste hiérarchique, un procédé d'estimation de concentrations moléculaires de protéines d'intérêt et d'aide au diagnostic mis en oeuvre par le processeur 28 par exécution de la séquence d'instructions 30 va maintenant être décrit.

Pour l'inversion, il existe trois conditions :

1) soit le gain d'ionisation $\xi$ est connu, alors on estimera $\alpha$ ;
2) soit la matrice de digestion $\alpha$ est connue, soit issue d'une connaissance a priori (base de données, expériences antérieures), soit issue d'un calibrage externe comme décrit ci-après (phases 200 et 400), soit connue d'après un modèle de monitoring faisant intervenir des paramètres physiques importants comme le pH ou la température de la solution de digestion ou la durée de la digestion, alors on estimera le gain d'ionisation $\xi$ ;

3) soit ni l'un ni l'autre n'est connu, alors suite à l'interchangeabilité des deux coefficients on n'estimera qu'un gain global équivalent que l'on notera $\xi'$.

Dans ce qui suit, nous allons d'abord décrire la théorie avec l'ensemble des paramètres. Pour se placer dans les cas précités, il convient :

- dans le cas 1), de supposer que $\xi = \xi_0$, i.e. la loi de probabilité pour $\xi$ est un Dirac centré en $\xi_0$;
- dans le cas 2), de supposer que $\alpha = \alpha_0$, i.e. la loi de probabilité pour $\alpha$ est un Dirac centré en $\alpha_0$ ;
- dans le cas 3), de supposer que $\alpha_{ip} = 1$ pour tout i et pour tout p, i.e. la loi de probabilité pour $\alpha$ est un Dirac centré en 1, ce qui revient à dire que le procédé d'inversion se chargera d'estimer le produit des variables qui est un gain global équivalent $\xi'$ et qui n'est pas séparable en raison de la nature des équations du modèle direct.

Le premier cas fait intervenir une estimation de la concentration des peptides K* issus des protéines marquées. Dans le troisième cas, le vecteur K n'est pas la concentration des peptides à proprement parler, mais un vecteur K' de concentration potentielle équivalente issue d'une digestion sans perte et sans discrimination des peptides mal digérés, K' = Dx et K'* = Dx*.

Selon ce procédé, l'estimation des concentrations moléculaires x se fait conjointement avec l'estimation de l'ensemble des variables aléatoires $\gamma_b$, $\xi$, T, K, K*, $\alpha$, x et B par application d'un estimateur sur la loi de probabilité a posteriori jointe de ces variables aléatoires au vu de l'observation Y. Cette loi de probabilité a posteriori jointe se développe de la façon suivante selon la règle de Bayes :

$$p(\gamma_b, \xi, T, K, K^*, \alpha, x, B | Y) = \frac{p(Y | \gamma_b, \xi, T, K, K^*, \alpha, x, B) p(\gamma_b, \xi, T, K, K^*, \alpha, x, B)}{p(Y)}. \quad (2)$$

Bien que la loi de vraisemblance $p(Y | \gamma_b, \xi, T, K, K^*, \alpha, x, B)$ puisse être exprimée analytiquement et bien que la loi jointe des paramètres $p(\gamma_b, \xi, T, K, K^*, \alpha, x, B)$ puisse se développer en un produit de lois de probabilités a priori conditionnelles modélisables par l'expérience ou par un calibrage spécifique, la loi marginale $p(Y)$ n'est pas connue et pas calculable analytiquement. Par conséquent la loi de probabilité a posteriori jointe n'est pas non plus calculable analytiquement puisque l'on ne connaît pas ce facteur multiplicatif $p(Y)$ qui cependant reste constant pour tous les paramètres. Ce facteur multiplicatif inconnu n'est donc pas pénalisant.

Il en résulte cependant que le calcul d'un estimateur, tel que l'estimateur espérance a posteriori, médiane a posteriori ou maximum a posteriori, sur cette loi a posteriori jointe ne peut se faire analytiquement de façon simple. C'est pourtant l'estimateur moyenne a posteriori sur les paramètres à lois de probabilités continues ($\gamma_b$, $\xi$, T, K, K*, $\alpha$, x) et maximum a posteriori sur le paramètre à loi de probabilité discrète (B) qu'il conviendrait d'appliquer.

Pour contourner cette impossibilité de calculer directement un tel estimateur sur la loi a posteriori jointe de l'équation (2), il est équivalent et avantageux de procéder à un échantillonnage stochastique numérique de chacun des paramètres $\gamma_b$, $\xi$, T, K, K*, $\alpha$, x et B selon la loi de probabilité a posteriori conditionnelle qu'il vérifie, conformément par exemple au procédé connu de Monte-Carlo à chaîne de Markov (procédé d'échantillonnage MCMC, de l'anglais « Markov Chain Monte Carlo »), procédé qui constitue une approximation d'un tirage aléatoire sous la loi a posteriori jointe. L'échantillonnage numérique MCMC peut être en particulier réalisé par des méthodes itératives du type échantillonnage stochastique de Gibbs impliquant éventuellement l'algorithme de Metropolis-Hastings et l'estimateur, par exemple espérance a posteriori, peut ensuite être approché simplement par les valeurs moyennes des échantillonnages respectifs.

On montre en effet qu'alors que la loi de probabilité a posteriori jointe de l'équation (2) n'est pas exprimable analytiquement à l'aide de probabilités a priori (conditionnelles ou non), c'est en revanche le cas des lois de probabilité a posteriori conditionnelles précitées, comme cela va maintenant être détaillé.

En particulier, compte tenu de la hiérarchie du modèle probabiliste de la figure 2, compte tenu également de la règle de Bayes et des marginalisations possibles de la loi de probabilité jointe des variables aléatoires impliquées, on montre aisément que la loi de probabilité a posteriori conditionnelle suivie par le paramètre de bruit $\gamma_b$ prend la forme suivante :

$p(\gamma_b | Y, \xi, T, K, K^*, \alpha, x, B) = p(\gamma_b | Y, \xi, T, K, K^*)$, puisque $\gamma_b$ peut être considéré comme indépendant a posteriori de $\alpha$, x et B, sa dépendance par rapport à ces paramètres passant par K et K*.

D'après la règle de Bayes appliquée au second membre de l'équation précédente :

$$p\left(\gamma_b \middle| Y, \xi, T, K, K^*, \alpha, x, B\right) = \frac{p(\gamma_b, Y, \xi, T, K, K^*)}{p(Y, \xi, T, K, K^*)} = \frac{p(\gamma_b, Y, \xi, T, K, K^*)}{\int p(\gamma_b, Y, \xi, T, K, K^*)d\gamma_b}$$

$$= \frac{p(\gamma_b)p(\xi)p(T)p(K)p(K^*)p(Y|\gamma_b, \xi, T, K, K^*)}{p(\xi)p(T)p(K)p(K^*)\int p(\gamma_b)p(Y|\gamma_b, \xi, T, K, K^*)d\gamma_b}$$

$$= p(Y|\gamma_b, \xi, T, K, K^*)p(\gamma_b)\frac{1}{\int p(\gamma_b)p(Y|\gamma_b, \xi, T, K, K^*)d\gamma_b}$$

$$\propto p(Y|\gamma_b, \xi, T, K, K^*)p(\gamma_b)$$

où $\propto$ est le symbole de proportionnalité, cette proportionnalité étant vérifiée puisque l'expression

$$\frac{1}{\int p(\gamma_b)p(Y|\gamma_b, \xi, T, K, K^*)d\gamma_b}$$ est un coefficient indépendant de $\gamma_b$.

On montre de la même façon que :

$$p\left(\xi \middle| Y, \gamma_b, T, K, K^*, \alpha, x, B\right) \propto p(Y|\gamma_b, \xi, T, K, K^*)p(\xi),$$

et

$$p\left(T \middle| Y, \gamma_b, \xi, K, K^*, \alpha, x, B\right) \propto p(Y|\gamma_b, \xi, T, K, K^*)p(T).$$

En ce qui concerne le paramètre K :

$p(K|Y, \gamma_b, \xi, T, \alpha, K^*, x, B) = p(K|Y, \gamma_b, \xi, T, \alpha, K^*, x)$, puisque K peut être considéré comme indépendant a posteriori de B, sa dépendance par rapport à ce paramètre passant par x.

D'après la règle de Bayes appliquée au second membre de l'équation précédente :

$$p\left(K \middle| Y, \gamma_b, \xi, T, \alpha, K^*, x, B\right) = \frac{p(\gamma_b, Y, \xi, T, K, K^*, \alpha, x)}{\int p(\gamma_b, Y, \xi, T, K, K^*, \alpha, x)dK}$$

$$= \frac{p(\gamma_b)p(\xi)p(T)p(K, K^*, \alpha, x)p(Y|\gamma_b, \xi, T, K, K^*, \alpha, x)}{p(\gamma_b)p(\xi)p(T)\int p(K, K^*, \alpha, x)p(Y|\gamma_b, \xi, T, K, K^*)dK}$$

$$= p(Y|\gamma_b, \xi, T, K, K^*)p(K|\alpha, x)\frac{p(K^*, \alpha, x)}{\int p(K, K^*, \alpha, x)p(Y|\gamma_b, \xi, T, K, K^*)dK}$$

$$= p(Y|\gamma_b, \xi, T, K, K^*)p(K|\alpha, x)\frac{1}{\int p(K|\alpha, x)p(Y|\gamma_b, \xi, T, K, K^*)dK}$$

$$\propto p(Y|\gamma_b, \xi, T, K, K^*)p(K|\alpha, x)$$

puisque l'expression $\dfrac{1}{\int p(K|\alpha,x)p(Y|\gamma_b,\xi,T,K,K*)dK}$ est un coefficient indépendant de K.

Par échange de K et K*, on en déduit aisément l'expression pour p(K* | Y, $\gamma_b$, $\xi$, T, $\alpha$, K, x, B) $\propto$ p(K* | Y, $\gamma_b$, $\xi$, T, K, $\alpha$) $\propto p(Y|\gamma_b,\xi,T,K,K^*)p(K^*|\alpha)$, la variable x* n'étant pas une variable aléatoire.

En ce qui concerne le paramètre $\alpha$ :

$p(\alpha|Y,\gamma_b,\xi,T,K,x,B)=p(\alpha|K,x)$, puisque $\alpha$ peut être considéré comme indépendant a posteriori de Y, $\gamma_b$, $\xi$, T et B, sa dépendance par rapport à ces paramètres passant par K et x.

D'après la règle de Bayes appliquée au second membre de l'équation précédente :

$$
\begin{aligned}
p(\alpha|Y,\gamma_b,\xi,T,K,K^*,x,B) &= \frac{p(K,K^*,\alpha,x)}{\int p(K,K^*,\alpha,x)d\alpha} \\
&= \frac{p(K|\alpha,x)p(K^*|\alpha)p(x)p(\alpha)}{\int p(K|\alpha,x)p(K^*|\alpha)p(x)p(\alpha)d\alpha} \\
&= \frac{p(K|\alpha,x)p(K^*|\alpha)p(x)p(\alpha)}{p(x)\int p(K|\alpha,x)p(K^*|\alpha)p(\alpha)d\alpha} \\
&= p(K|\alpha,x)p(K^*|\alpha)p(\alpha)\frac{1}{\int p(K|\alpha,x)p(K^*|\alpha)p(\alpha)d\alpha} \\
&\propto p(K|\alpha,x)p(K^*|\alpha)p(\alpha)
\end{aligned}
$$
,

puisque l'expression $\dfrac{1}{\int p(K|\alpha,x)p(K^*|\alpha)p(\alpha)d\alpha}$ est un coefficient indépendant de $\alpha$.

En ce qui concerne le paramètre x :

$p(x|Y,\gamma_b,\xi,T,K,K^*\alpha,B)=p(x|K,\alpha,B)$, puisque x peut être considéré comme indépendant a posteriori de Y, $\gamma_b$, $\xi$, K* et T, sa dépendance par rapport à ces paramètres passant par K.

D'après la règle de Bayes appliquée au second membre de l'équation précédente et en notant Pr(B) la probabilité à valeurs discrètes du paramètre B :

$$
\begin{aligned}
p(x|Y,\gamma_b,\xi,T,K,K^*,\alpha,B) &= \frac{p(K,\alpha,x,B)}{\int p(K,\alpha,x,B)dx} \\
&= \frac{p(K|\alpha,x,B)p(x|\alpha,B)p(\alpha)}{\int p(K|\alpha,x,B)p(x|\alpha,B)p(\alpha)dx} \\
&= \frac{p(K|\alpha,x)p(x|B)\Pr(B)p(\alpha)}{\Pr(B)p(\alpha)\int p(K|\alpha,x)p(x|B)dx} \\
&= p(K|\alpha,x)p(x|B)\frac{1}{\int p(K|\alpha,x)p(x|B)dx} \\
&\propto p(K|\alpha,x)p(x|B)
\end{aligned}
$$
,

puisque l'expression $\dfrac{1}{\int p(K|\alpha,x)p(x|B)dx}$ est un coefficient indépendant de x.

Enfin, en ce qui concerne le paramètre B :

$Pr(B|Y,\gamma_b,\xi,T,K,K^*,\alpha,x)=Pr(B|x)$, puisque B peut être considéré comme indépendant a posteriori de Y, $\gamma_b$, $\xi$, T, K, K$^*$ et $\alpha$, sa dépendance par rapport à ces paramètres passant par x.

D'après la règle de Bayes appliquée au second membre de l'équation précédente :

$$\Pr(B|Y,\gamma_b,\xi,T,K,K^*,\alpha,x) = \frac{p(x|B)\Pr(B)}{p(x)}$$
$$\propto p(x|B)\Pr(B)$$

puisque l'expression $\dfrac{1}{p(x)}$ est un coefficient indépendant de B.

En résumé :

$$p(\gamma_b|Y,\xi,T,K,K^*,\alpha,x,B) = p(\gamma_b|Y,\xi,T,K,K^*) \propto p(Y|\gamma_b,\xi,T,K,K^*)p(\gamma_b), \qquad (3)$$

$$p(\xi|Y,\gamma_b,T,K,K^*,\alpha,x,B) = p(\xi|Y,\gamma_b,T,K,K^*) \propto p(Y|\gamma_b,\xi,T,K,K^*)p(\xi), \qquad (4)$$

$$p(T|Y,\gamma_b,\xi,K,K^*,\alpha,x,B) = p(T|Y,\gamma_b,\xi,K,K^*) \propto p(Y|\gamma_b,\xi,T,K,K^*)p(T), \qquad (5)$$

$$p(K|Y,\gamma_b,\xi,T,\alpha,K^*,x,B) = p(K|Y,\gamma_b,\xi,T,K^*,\alpha,x)$$
$$\propto p(Y|\gamma_b,\xi,T,K,K^*)p(K|\alpha,x)' \qquad (6)$$

$$p(K^*|Y,\gamma_b,\xi,T,K,\alpha,B) = p(K^*|Y,\gamma_b,\xi,T,K,\alpha)$$
$$\propto p(Y|\gamma_b,\xi,T,K,K^*)p(K^*|\alpha)' \qquad (6^*)$$

$$p(\alpha|Y,\gamma_b,\xi,T,K,K^*,x,B) = p(\alpha|K,K^*,x) \propto p(K|\alpha,x)p(K^*|\alpha,x)p(\alpha), \qquad (7)$$

$$p(x|Y,\gamma_b,\xi,T,K,K^*,\alpha,B) = p(x|K,\alpha,B) \propto p(K|\alpha,x)p(x|B), \text{ et} \qquad (8)$$

$$\Pr(B|Y,\gamma_b,\xi,T,K,K^*,\alpha,x) = \Pr(B|x) \propto p(x|B)\Pr(B). \qquad (9)$$

L'équation (6*) n'est utile que dans le cas précité 1) suite à l'estimation de la matrice de digestion $\alpha$.
Il est à noter que lorsque l'on se trouve dans les cas précités, il convient d'omettre l'écriture du paramètre constant ($\xi$ dans le cas n° 1 ou $\alpha$ dans les cas n° 2 et 3) de manière à ce que les dépendances ne l'incluent pas.

Dans les cas 2) et 3), le paramètre K* est également connu, car $\alpha$ est soit connu (cas n° 2), soit supposé égal à 1 (cas n° 3). K* n'intervient donc pas dans les lois. Ainsi, dans le cas 3), l'équation (6) s'écrira p(K' Y, $\gamma_b$, $\xi$', T, x, B) = p(K' | Y, $\gamma_b$, $\xi$ ', T, x).

On remarque que dans le cas particulier où le paramètre K est défini de manière déterministe à partir de x (digestion parfaite et déterministe ou déterministe avec $\alpha$ connu), il n'y a plus d'incertitude sur ce paramètre. Cela se traduit par le jeu d'équations simplifié suivant :

$$p(\gamma_b|Y,\xi,T,\alpha,x,B) = p(\gamma_b|Y,\xi,T,x) \propto p(Y|\gamma_b,\xi,T,x)p(\gamma_b), \qquad (10)$$

$$p(\xi|Y,\gamma_b,T,x,B) = p(\xi|Y,\gamma_b,T,x) \propto p(Y|\gamma_b,\xi,T,x)p(\xi), \qquad (11)$$

$$p(T|Y,\gamma_b,\xi,x,B) = p(T|Y,\gamma_b,\xi,x) \propto p(Y|\gamma_b,\xi,T,x)p(T), \qquad (12)$$

$$p(x|Y,\gamma_b,\xi,T,B) = p(x|Y,\gamma_b,\xi,T) \propto p(Y|\gamma_b,\xi,T,K)p(x|B), \text{ et} \qquad (13)$$

$$\Pr(B|Y,\gamma_b,\xi,T,x) = \Pr(B|x) \propto p(x|B)\Pr(B). \qquad (14)$$

Les équations (10) à (13) se ramènent à celles développées dans la demande de brevet EP 2 028 486 à la différence près qu'un étage de hiérarchie est ajouté (ajout de la loi a priori conditionnelle $p(x|B)$).

On peut aussi ne pas avoir de paramètre d'état biologique. Dans ce cas, B est déterminé à l'avance, l'équation (9) perd son intérêt et les équations (3) à (8) ne contiennent plus la variable B.

On peut aussi avoir un paramètre d'état biologique et une digestion déterministe.

Les équations (3) à (9) montrent que les lois de probabilité a posteriori conditionnelles de tous les paramètres $\gamma_b$, $\xi$, T, K, K*, $\alpha$, x et B sont exprimables analytiquement puisqu'elles sont proportionnelles à des produits de distributions ou probabilités a priori qui sont soit modélisables soit définissables par apprentissage.

Notamment, on montre, comme cela peut être déduit aisément du document EP 2028486, que la fonction de vraisemblance $p(Y|\gamma_b,\xi,T,K,K*)$ suit une loi normale de moyenne HK+H*K* et d'inverse variance $\gamma_b$, où H et H* correspondent à une réécriture de l'équation (1) sous la forme $Y = HK + H*K*+b(\gamma_b)$.

Pour le bruit $\gamma_b$, on choisit un modèle de probabilité a priori $p(\gamma_b)$ de type loi Gamma de paramètres $\alpha_G$ et $\beta_G$. En particulier, lorsque $\alpha_G$ tend vers 0 et $\beta_G$ vers l'infini, cette loi Gamma devient une loi de Jeffreys (ce qui traduit alors l'absence d'a priori que l'on a sur le bruit de mesure).

Pour le temps de rétention T, on choisit un modèle de probabilité a priori $p(T)$ de type loi uniforme entre un vecteur Tmin de valeurs minimales pour chaque peptide i et un vecteur Tmax de valeurs maximale pour chaque peptide i.

Dans les trois cas de réalisation précités, on procède de la manière suivante :

1) - pour le gain $\xi$, ayant connaissance de sa valeur nominale $\xi_0$, on choisit une distribution de Dirac centrée en $\xi_0$ pour modéliser sa probabilité a priori p($\xi$) et a posteriori p($\xi$ | y, K, K*, T, $\gamma_b$),

- pour le coefficient de digestion $\alpha$, on choisit un modèle de probabilité de type loi uniforme dans un intervalle compris entre 0 et 1,
- pour la concentration des peptides K, étant donné $\xi_0$ et la relation entre K et x (et K* et x*) déterminée par la matrice de digestion $\alpha$D, avec un éventuel bruit blanc gaussien, le modèle de probabilité a priori conditionnelle $p(K|\alpha,x)$ est de type loi normale de moyenne $\mu_{K|x}$ et d'inverse-matrice de covariance $\Gamma_{K|x} = R_{K|x}^{-1}$,
- idem pour p(K* | $\alpha$) ;

2) - pour le gain $\xi$, on choisit un modèle de probabilité a priori $p(\xi)$ de type loi normale de moyenne $\mu_\xi$ et d'inverse-matrice de covariance $\Gamma_\xi = R_\xi^{-1}$. Etant donné que le paramètre de gain $\xi$ est en relation linéaire avec l'observation Y, $Y = G\xi+b(\gamma_b)$, on notera que l'on peut alors considérer que la fonction de vraisemblance

$p(Y|\gamma_b,\xi,T,K,K^*)=p(Y|\gamma_b,\xi,T,K)$ suit une loi normale de moyenne $G\xi$ = HK+H*K* et d'inverse variance $\gamma_b$,

- pour le coefficient de digestion $\alpha$, en supposant connue la valeur nominale constante $\alpha_0$, on choisit une distribution de Dirac centrée en $\alpha$ pour modéliser sa probabilité a priori $p(\alpha)$ et a posteriori $p(\alpha|Y,\gamma_b,\xi,T,K,K^*,x,B)=p(\alpha|K,K^*,x)$,
- pour la concentration des peptides K, étant donné $\xi$ et la relation entre K et x déterminée par la matrice de digestion $\alpha_0 D$, avec un éventuel bruit blanc gaussien, le modèle de probabilité a priori conditionnelle $p(K|\alpha,x)=p(K|x)$ est de type loi normale de moyenne $\mu_{K|x}$ et d'inverse-matrice de covariance $\Gamma_{K|x} = R_K|x^{-1}$ ;

3) dans l'ignorance des valeurs nominales des deux paramètres $\alpha$ et $\xi$, il convient d'estimer un gain global $\xi'$. Pour cela, on choisit pour $\alpha$ une distribution de Dirac centrée en 1 pour ses lois a priori et a posteriori,

- pour le gain global $\xi'$, on choisit un modèle de probabilité a priori $p(\xi')$ de type loi normale de moyenne $\mu\xi'$ et d'inverse-matrice de covariance $\Gamma_{\xi'} = R_{\xi'}^{-1}$. Étant donné que le paramètre de gain $\xi'$ est en relation linéaire avec l'observation Y, $Y = G\xi' + b(\gamma_b)$, on notera que l'on peut alors considérer que la fonction de vraisemblance p(Y | $\gamma_b$, $\xi'$, T, K') suit une loi normale de moyenne $G\xi'$ = HK' + H* K'* et d'inverse variance $\gamma_b$,
- pour la concentration potentielle des peptides K', étant donné le gain global $\xi'$ et la relation entre K' et x déterminée par la matrice de digestion D, avec un éventuel bruit blanc gaussien, le modèle de probabilité a priori conditionnelle $p(K'|\alpha,x)=p(K'|x)$ est de type loi normale de moyenne $\mu_{K'|x}$ et d'inverse-matrice de covariance $\Gamma_{K'|x} = R_{K'|x}^{-1}$.

Pour la concentration des protéines d'intérêt x, on choisit un modèle de probabilité a priori conditionnelle $p(x|B)$ de type loi normale de moyenne $\mu_{x|B}$ et d'inverse-matrice de covariance $\Gamma_{x|B} = R_{x|B}^{-1}$. Les moyenne et inverse-matrice de covariance de la concentration x pour chacun des états B possibles sont par exemple déterminées par apprentissage sur des ensembles d'échantillons dont l'état B correspondant est connu a priori.

Pour l'état B, la probabilité a priori Pr(B) est à valeurs discrètes. Si l'on considère par exemple deux états possibles, un état sain S et un état pathologique P, il est possible de fixer a priori deux valeurs $p_S$ et $p_P$ comprises entre 0 et 1 et telles que $p_S + p_P = 1$.

Au vu des probabilités a priori détaillées ci-dessus et de l'équation (3), il vient que la probabilité a posteriori pour le bruit $\gamma_b$ s'exprime sous la forme d'une loi Gamma multipliée par la fonction de vraisemblance qui est sous la forme d'une loi normale. La famille des lois Gamma étant conjuguée par celle des lois normales, on trouve alors que cette probabilité a posteriori $p(\gamma_b|Y,\xi,T,K)$ suit elle-même une loi Gamma de paramètres $\alpha'_G$ et $\beta'_G$ de valeurs suivantes :

$$\alpha_G^{'} = \alpha + N/2,$$

et

$$\beta_G^{'} = \left( \beta_G^{-1} + \frac{\left\| Y - H^T Y - H^{*T} H * K * \right\|^2}{2} \right)^{-1},$$

où N est le nombre de pixels du chromatospectrogramme Y et $\alpha_G$ et $\beta_G$ sont les hyperparamètres a priori. Idem pour le cas 3) précité avec les paramètres $\xi'$ et K'..

L'échantillonnage de l'inverse variance $\gamma_b$ du bruit b pourra ainsi se faire simplement dans le cadre d'un procédé itératif d'échantillonnage de Gibbs.

Au vu des probabilités a priori détaillées ci-dessus et de l'équation (4), il vient que la probabilité a posteriori pour le gain $\xi$ dans le cas 2) s'exprime sous la forme d'une loi normale multipliée par la fonction de vraisemblance qui est elle-même normale. La famille des lois normales étant autoconjuguée, on trouve alors que cette probabilité a posteriori $p(\xi|Y,\gamma_b,T,K)$ suit aussi une loi normale de moyenne $\mu'_\xi$ et d'inverse-matrice de covariance $\Gamma'_\xi$ vérifiant les équations suivantes :

$$\mu_\xi^{'} = \Gamma_\xi^{'-1}\left(\Gamma_\xi\mu_\xi + \gamma_b G^T Y\right),$$

et

$$\Gamma'_\xi = \Gamma_\xi + \gamma_b G^T G .$$

Pour ne pas supposer de valeur connue du gain du système et rendre sa loi de probabilité a priori non informative, on peut supposer $\Gamma_\xi = 0$, d'où :

$$\mu'_\xi = \left(G^T G\right)^{-1} G^T Y ,$$

et

$$\Gamma'_\xi = \gamma_b G^T G .$$

L'échantillonnage du gain $\xi$ pourra ainsi se faire simplement dans le cadre d'un procédé itératif d'échantillonnage de Gibbs.
Il en est de même dans le cas 3) précédemment exposé pour la loi a posteriori conditionnelle de $\xi'$ en prenant soin d'utiliser K'. Dans le cas 1), il vient que la probabilité a posteriori pour $\xi$ s'exprime sous la forme d'une distribution de Dirac centrée en $\xi_0$.
Au vu des probabilités a priori détaillées ci-dessus et de l'équation (5), il vient que la probabilité a posteriori pour le temps de rétention T s'exprime sous la forme du produit d'une loi uniforme et de la fonction de vraisemblance. Cette probabilité a posteriori $p(T|Y,\gamma_b,\xi,K,K^*)$, ou $p(T \mid Y, \gamma_b, \xi', K')$ dans le cas n°3, n'a pas d'expression simple, de sorte que l'échantillonnage du temps de rétention T ne pourra pas se faire simplement dans le cadre d'un procédé itératif d'échantillonnage de Gibbs. Il sera nécessaire d'utiliser une technique d'échantillonnage telle que l'algorithme de Metropolis-Hastings par exemple.
Au vu des probabilités a priori détaillées ci-dessus et de l'équation (6), il vient que la probabilité a posteriori pour la concentration des peptides K (ou K' dans le cas n° 3) s'exprime sous la forme d'une loi normale multipliée par la fonction de vraisemblance qui est elle-même normale. On trouve alors par conjugaison des lois que cette probabilité a posteriori $p(K|Y, \gamma_b,\xi,T,\alpha,x,K^*)$ (ou $p(K' \mid Y, \gamma_b, \xi', T, x)$ dans le cas n°3) suit aussi une loi normale de moyenne $\mu'_K$ et d'inverse-matrice de covariance $\Gamma'_K$ (voire $\mu'_{K'}$ et $\Gamma'_{K'}$ dans le cas n°3) vérifiant les équations suivantes :

$$\mu'_K = \Gamma'_K{}^{-1}\left(\Gamma_{K|x}\mu_{K|x} + \gamma_b\left(H^T Y + H^{*T} H * K *\right)\right),$$

et

$$\Gamma'_K = \Gamma_{K|x} + \gamma_b H^T H .$$

Dans le cas n°3, on remplace, dans les équations ci-dessus, K par K'.
L'échantillonnage de la concentration des peptides K (voire K' dans le cas n° 3) pourra ainsi se faire simplement dans le cadre d'un procédé itératif d'échantillonnage de Gibbs.
Il en est de même pour la concentration des peptides K* issus de la protéine marquée de concentration x* qui suit un raisonnement analogue au précédent dans le cas 1).
Au vu des probabilités a priori détaillées ci-dessus, de l'équation (7) et des cas précités, il vient que la probabilité a posteriori $p(\alpha|K, K^*, x)$ pour le coefficient de digestion $\alpha$ s'exprime sous la forme d'une distribution de Dirac centrée en $\alpha_0$ dans le cas 2) et sous la forme d'une distribution de Dirac centrée en 1 dans le cas 3). L'échantillonnage de ce paramètre dans l'exemple considéré est donc trivial. Dans le cas 1), la loi a posteriori $p(\alpha|K,K^*,x)$ n'a pas d'expression simple, de sorte que l'échantillonnage du coefficient de digestion $\alpha$ ne pourra se faire simplement dans le cadre d'un procédé itératif d'échantillonnage de Gibbs. Il sera nécessaire d'utiliser une technique d'échantillonnage telle que l'algorithme de Metropolis-Hasting par exemple.

Au vu des probabilités a priori détaillées ci-dessus et de l'équation (8), il vient que la probabilité a posteriori $p(x|k,\alpha,B)$ (voire p(x | K', B) dans le cas n° 3) pour la concentration des protéines d'intérêt x s'exprime, comme pour les peptides, par autoconjugaison de la loi a priori avec la fonction de vraisemblance sous la forme d'une loi normale de moyenne $\mu'_x$ et d'inverse-matrice de covariance $\Gamma'_x$ vérifiant les équations suivantes :

$$\mu'_x = \Gamma'^{-1}_x \left( \Gamma_{K|x} K^T D + \Gamma_{x|B} \mu_{x|B} \right),$$

et

$$\Gamma'_x = \Gamma_{x|B} + \Gamma_{K|x} D^T D .$$

L'échantillonnage de la concentration des protéines d'intérêt x pourra ainsi se faire simplement dans le cadre d'un procédé itératif d'échantillonnage de Gibbs.

Au vu des probabilités a priori détaillées ci-dessus et de l'équation (9), il vient que la probabilité a posteriori $\Pr(B|x)$ pour l'état biologique B s'exprime de la façon suivante, sur l'hypothèse des deux états précités S et P :

$$\Pr(B|x) = \frac{p(x|B)\Pr(B)}{p(x)}$$

$$= \frac{\Pr(B)\sqrt{\det(\Gamma_{x|B})}\exp\left(-\frac{1}{2}(x-\mu_{x|B})^T \Gamma_{x|B}(x-\mu_{x|B})\right)}{p_S\sqrt{\det(\Gamma_{x|S})}\exp\left(-\frac{1}{2}(x-\mu_{x|s})^T \Gamma_{x|s}(x-\mu_{x|s})\right) + p_P\sqrt{\det(\Gamma_{x|P})}\exp\left(-\frac{1}{2}(x-\mu_{x|P})^T \Gamma_{x|P}(x-\mu_{x|P})\right)}$$

L'échantillonnage de l'état biologique B pourra ainsi se faire simplement dans le cadre d'un procédé itératif d'échantillonnage de Gibbs.

Pour les paramètres dont la probabilité a posteriori conditionnelle suit une loi normale, c'est-à-dire les paramètres

1) $\alpha$, K, K*, x
2) $\xi$, K, x
3) $\xi'$, K', x

selon le cas considéré, l'échantillonnage dans le cadre du procédé itératif de Gibbs suit un algorithme A.

ALGORITHME A (pour un vecteur x de taille n suivant une loi normale de moyenne $\mu$ et d'inverse-matrice de covariance $\Gamma$) :

- Calcul de la matrice de covariance $R = \Gamma^{-1}$,
- Calcul de la décomposition de Cholesky de la matrice de covariance $R = \Lambda^T \Lambda$,
- Génération d'un vecteur v de n variables indépendantes distribuées suivant une loi normale centrée réduite,
- Calcul de l'échantillon $x = \mu + \Lambda^T v$.

Pour le paramètre $\gamma_b$ dont la probabilité a posteriori conditionnelle suit une loi Gamma, l'échantillonnage dans le cadre du procédé itératif de Gibbs suit un algorithme B.

ALGORITHME B (pour un vecteur x de taille N) :

- Calcul de $\alpha_G = \alpha_G + N/2$,

- Calcul de $\beta_G' = \left( \beta_G^{-1} + \dfrac{\chi(K, K^*, \xi, \gamma_b, T)}{2} \right)^{-1}$,

- Génération d'une variable aléatoire suivant la loi Gamma avec les paramètres $\alpha'_G$ et $\beta'_G$,

où

$$\chi(K, K^*, \xi, \gamma_b, T) = \left\| Y - \sum_{i=1}^{I} \sum_{j=1}^{J} \sum_{K=1}^{K} \xi_i \pi_{ij} \left( K_i \pi'_{ijk} s_{ijk} + K_i^* \pi'^*_{ijk} s^*_{ijk} \right) c_i^T (T_i) \right\|^2.$$

Remarque : dans le cas 3), en remplacera par $\xi'$, K par K' et K* par K'*.

Pour le paramètre T dont la probabilité a posteriori conditionnelle n'a pas d'expression simple, l'échantillonnage dans le cadre du procédé itératif de Gibbs suit un algorithme C mettant en oeuvre l'algorithme de Metropolis-Hastings avec Marche Aléatoire (MHMA).

ALGORITHME C (pour la génération de T à l'itération k+1, noté $T^{(k+1)}$) :

- Générer un vecteur de valeurs proposées $T^P$ selon la loi normale de moyenne $T^{(k)}$ et d'inverse-matrice de covariance $\Gamma_{MHMA}$, où $\Gamma_{MHMA}$ pilote la génération de variables aléatoires,
- Générer une valeur u selon la loi uniforme sur l'intervalle [0,1],
- Calculer :

$$\delta = \exp\left( -\frac{\gamma_b}{2} \cdot \left( \chi\left( K^{(k)}, K^{*(k)}, \xi^{(k+1)}, \gamma_b^{(k+1)}, T^P \right) - \chi\left( K^{(k)}, K^{*(k)}, \xi^{(k+1)}, \gamma_b^{(k+1)}, T^{(k)} \right) \right) \right),$$

- Si $\delta > u$ alors $T^{(k+1)} = T^P$, sinon $T^{(k+1)} = T^{(k)}$.

Remarque : dans le cas 3), en remplacera par $\xi'$, K par K' et K* par K'*.

Pour le paramètre B, l'échantillonnage dans le cadre du procédé itératif de Gibbs suit un algorithme D.

ALGORITHME D (pour la génération de B à l'itération k, noté $B^{(k)}$ lorsque B est à deux états possibles S et P) :

- Générer une valeur u selon la loi uniforme sur l'intervalle [0,1],
- Si $u \in [0, p_S]$ alors $B^{(k)} = S$ et $p_B^{(k)} = [1,0]$, sinon $B^{(k)} = P$ et $p_B^{(k)} = [0,1]$.

Enfin, pour le paramètre $\alpha$, dans le cas 1) dont la probabilité a posteriori conditionnelle n'a pas d'expression simple, l'échantillonnage dans le cadre du procédé itératif de Gibbs suit un algorithme E mettant en oeuvre l'algorithme de Metropolis-Hasting avec Marche Aléatoire (MHMA)..

ALGORITHME E (pour la génération de $\alpha$ à l'itération k+1, noté $\alpha^{(k+1)}$) :

- Générer un vecteur de valeurs proposées $\alpha^P$ selon la loi normale de moyenne $\alpha^{(k)}$ et d'inverse-matrice de covariance $\Gamma_{MHMA}$, ne sortant pas de l'intervalle [0, 1], où $\Gamma_{MHMA}$ pilote la génération de variables aléatoires,
- Générer une valeur u selon la loi uniforme sur l'intervalle [0, 1],

- Calculer $\delta = \dfrac{p(K | \alpha^P, x) p(K^* | \alpha^P)}{p(K | \alpha^{(k)}, x) p(K^* | \alpha^{(k)})}$,

- Si $\delta > u$ alors $\alpha^{(k+1)} = \alpha^P$, sinon $\alpha^{(k+1)} = \alpha^{(k)}$.

Au vu de ce qui précède et en référence à la figure 3, un procédé d'estimation de paramètres biologiques ou chimiques, ou plus précisément, dans le cadre de l'exemple non limitatif choisi, un procédé d'estimation de concentrations moléculaires de protéines d'intérêt et d'aide au diagnostic mis en oeuvre par le processeur 28 par exécution de la séquence d'instructions 30 comporte une phase principale 100 d'estimation conjointe des paramètres dits incertains d'un échantillon biologique E dont on ne connaît pas l'état biologique, dont notamment au moins l'un des deux paramètres suivants : le vecteur x des concentrations en protéines d'intérêt et l'état biologique B.

L'exécution de cette phase principale d'estimation conjointe 100 suppose qu'un certain nombre de données et paramètres soient déjà connus et enregistrés dans la base de données 32, à savoir :

- les paramètres dits certains ou constants de la chaîne de traitement biologique 12, c'est-à-dire ceux qui sont invariants d'un traitement biologique à l'autre,
- une sélection établie et identifiée de composants d'intérêt, en l'occurrence dans le cadre de l'exemple non limitatif choisi, une sélection de protéines d'intérêt à partir de laquelle peut être défini le vecteur x des concentrations en protéines d'intérêt, et
- les paramètres des modèles de probabilités a priori, conditionnelles ou non, des paramètres incertains à partir desquels peuvent être déterminés les modèles de probabilités a posteriori conditionnelles de chacun de ces paramètres.

Lorsqu'au moins une partie de ces données n'est pas connue, le procédé d'estimation de paramètres biologiques ou chimiques et d'aide au diagnostic peut être optionnellement complété de l'une ou plusieurs des phases préalables suivantes :

- une première phase 200 de calibrage, dit calibrage externe, de la chaîne de traitement 12 réalisée à l'aide d'au moins un échantillon $E_{CALIB1}$ de composants de calibrage externe dont la concentration est connue, par exemple un échantillon de protéines standard ou de protéines prédéterminées contenues dans des cocktails de protéines, la concentration moléculaire de ces protéines de calibrage étant connue :

  ■ pour la détermination des paramètres certains non encore connus et leur enregistrement dans la base de données 32, et/ou
  ■ pour la détermination de paramètres stables (par exemple moyenne ou variance) des modèles de probabilités a priori des paramètres incertains et leur enregistrement dans la base de données 32,

  (Dans les cas 1 et 2, ce calibrage externe 200 peut permettre de déterminer respectivement un paramètre de la loi de probabilité de $\alpha$ (par exemple moyenne ou covariance, min, max, ...) ou la valeur moyenne de $\alpha$)
- une phase 300 de sélection de composants d'intérêt, par exemple de protéines d'intérêt dans le cadre de l'exemple non limitatif illustré, réalisée à l'aide d'un ensemble $E_{REF}$ d'échantillons dits de référence parce que leurs états biologiques respectifs sont connus, permettant de sélectionner, en tant que protéines d'intérêt, les protéines dont les concentrations sont les plus discriminantes par rapport aux états biologiques possibles,
- une seconde phase optionnelle 400 de calibrage externe de la chaîne de traitement 12 réalisée à l'aide d'au moins un échantillon $E_{CALIB2}$ de composants choisis parmi les composants d'intérêt et dont la concentration est connue, par exemple un échantillon de protéines d'intérêt dont la concentration moléculaire est connue :

  ■ pour une détermination plus fine des paramètres certains et leur mise à jour dans la base de données 32, et/ou
  ■ pour une détermination plus fine de paramètres stables (par exemple moyenne ou variance) des modèles de probabilités a priori des paramètres incertains et leur mise à jour dans la base de données 32, (Dans les cas 1 et 2, ce calibrage externe 400 peut permettre de déterminer respectivement un paramètre de la loi de probabilité de $\alpha$ (par exemple moyenne ou covariance, min, max, ...) ou la valeur moyenne de $\alpha$)

- une phase 500 d'apprentissage d'au moins une partie des modèles de probabilité a priori des paramètres incertains, cette phase étant réalisée à l'aide de l'ensemble d'échantillons $E_{REF}$ et d'au moins un échantillon $E^*$ de composants (dans l'exemple choisi, ce sont des protéines) de marquage (équivalents aux composants d'intérêt mais de masse différente), pour la détermination des paramètres de ces modèles et leur enregistrement dans la base de données 32.

Les phases 200 et 300 peuvent être qualifiées de phases de découverte, parce qu'exécutées alors qu'aucun composant particulier d'intérêt n'est sélectionné, tandis que les phases 400, 500 et 100 peuvent être qualifiées de phases de validation, parce qu'exécutées en s'intéressant spécifiquement aux paramètres de composants d'intérêt bien identifiés.

Lorsque les cinq phases précitées sont appliquées, il convient de les exécuter dans l'ordre suivant : (1) la première phase de calibrage externe 200 pour la détermination des paramètres certains non encore connus et des paramètres statistiques stables à l'aide de l'échantillon $E_{CALIB1}$ et leur enregistrement dans la base 32, puis (2) la phase de sélection 300 pour la détermination des composants d'intérêt à l'aide de l'ensemble d'échantillons $E_{REF}$, des paramètres certains et des paramètres statistiques stables et leur enregistrement dans la base 32, puis (3) la seconde phase optionnelle de calibrage externe 400 pour la détermination affinée des paramètres certains et des paramètres statistiques stables à l'aide de l'échantillon $E_{CALIB2}$, ce dernier pouvant comporter des composants de marquage, et leur mise à jour dans la base 32, puis (4) la phase d'apprentissage 500 pour la détermination d'au moins une partie des modèles de probabilité a priori des paramètres incertains, à l'aide des échantillons E* et $E_{REF}$, des paramètres certains et de la sélection identifiée de composants d'intérêt, et leur enregistrement dans la base 32, puis (5) la phase principale d'estimation conjointe 100 pour l'estimation des paramètres biologiques ou chimiques (i.e. les concentrations moléculaires des protéines d'intérêt dans l'exemple choisi) et une aide au diagnostic à l'aide des échantillons E, E* et des données enregistrées précédemment dans la base de données 32.

On notera que les phases 500 et 100 utilisent l'échantillon E* de composants de marquage, de sorte qu'elles peuvent être considérées comme incluant un calibrage interne pour une estimation des paramètres biologiques ou chimiques précise quantitativement. Il peut également en être de même pour la seconde phase optionnelle 400.

Les phases 100 à 500 vont maintenant être détaillées dans le cadre de l'exemple choisi d'une analyse biologique d'un échantillon biologique dont les paramètres biologiques comportent des concentrations moléculaires de protéines d'intérêt, mais elles sont généralisables comme indiqué précédemment.

La phase principale d'estimation conjointe 100 comporte une première étape de mesure 102 lors de laquelle, conformément au montage de la figure 1, l'échantillon E auquel on a ajouté les protéines de marquage E* traverse toute la chaîne de traitement 12 du dispositif 10 pour la fourniture d'un chromatospectrogramme Y.

Ensuite, lors d'une étape d'initialisation 104, les variables aléatoires $\gamma_b$, $\xi$, T, K, $\alpha$, x et B sont chacune initialisées par le processeur 28 à une première valeur $\gamma_b^{(0)}$ $\xi^{(0)}$, $T^{(0)}$, $K^{(0)}$, $\alpha^{(0)}$, $x^{(0)}$ et $B^{(0)}$.

Le processeur 28 exécute alors, par application d'un algorithme de Monte-Carlo à chaîne de Markov et sur un indice k variant de 1 à kmax, une boucle 106 d'échantillonnage de Gibbs de chacune des variables aléatoires initialisées au vu de leurs lois de probabilité a posteriori conditionnelles respectives telles qu'exprimées analytiquement. kmax est la valeur maximale prise par l'indice k avant qu'un critère d'arrêt prédéterminé ne soit vérifié. Le critère d'arrêt est par exemple un nombre d'itérations maximal fixé a priori, la satisfaction d'un critère de stabilité (tel que le fait qu'une itération supplémentaire n'a pas d'impact significatif sur l'estimateur choisi des variables aléatoires) ou autre.

Plus précisément, pour k variant de 1 à kmax, la boucle 106 comporte les échantillonnages successifs suivants :

Dans le cas 1) :

- générer un échantillon $\gamma_b^{(k)}$ à partir de la loi a posteriori $p(\gamma_b|Y,\xi^{(k)},T^{(k-1)},K^{(k-1)},K^{*(k-1)})$ conformément à l'algorithme B,
- générer un échantillon $T^{(k)}$ à partir de la loi a posteriori $p(T|Y,\gamma_b^{(k)},\xi^{(k)},K^{(k-1)},K^{*(k-1)})$ conformément à l'algorithme C,
- générer un échantillon $K^{(k)}$ à partir de la loi a posteriori $p(K|Y,\gamma_b,^{(k)},\xi^{(k)},T^{(k)},K^{*(k-1)},\alpha^{(k-1)},x^{(k-1)})$ conformément à l'algorithme A,
- générer un échantillon $K^{*(k)}$ à partir de la loi a posteriori $p(K*|Y,\gamma_b^{(k)},\xi^{(k)},T^{(k)},K^{(k)},\alpha^{(k-1)},x^{(k-1)})$ conformément à l'algorithme A,
  et, du fait de la structure hiérarchique du modèle tel qu'illustré par la figure 2 :

  - générer un échantillon $a^{(k)}$ à partir de la loi a posteriori $p(\alpha|K^{(k)},K^{*(k)},x^{(k-1)})$ conformément à l'algorithme E,
  - générer un échantillon $x^{(k)}$ à partir de la loi a posteriori $p(x|K^{(k)},\alpha^{(k)},B^{(k-1)})$ conformément à l'algorithme A,
  - générer un échantillon $B^{(k)}$ à partir de la loi a posteriori $Pr(B|x^{(k)})$ conformément à l'algorithme D.

Dans le cas 2) :

- générer un échantillon $\xi^{(k)}$ à partir de la loi a posteriori $p(\xi|Y,\gamma_b^{(k-1)},T^{(k-1)},K^{(k-1)})$ conformément à l'algorithme A,
- générer un échantillon $\gamma_b^{(k)}$ à partir de la loi a posteriori $p(\gamma_b|Y,\xi^{(k)},T^{(k-1)},K^{(k-1)})$ conformément à l'algorithme B,
- générer un échantillon $T^{(k)}$ à partir de la loi a posteriori $p(T|Y,\gamma_b^{(k)},\xi^{(k)},K^{(k-1)})$ conformément à l'algorithme C,
- générer un échantillon $K^{(k)}$ à partir de la loi a posteriori $p(K|Y,\gamma_b^{(k)},\xi^{(k)},T^{(k)},x^{(k-1)})$ conformément à l'algorithme A,
  et, du fait de la structure hiérarchique du modèle tel qu'illustré par la figure 2 :

  - générer un échantillon $x^{(k)}$ à partir de la loi a posteriori $p(x|K^{(k)},B^{(k-1)})$ conformément à l'algorithme A,
  - générer un échantillon $B^{(k)}$ à partir de la loi a posteriori $Pr(B|x^{(k)})$ conformément à l'algorithme D,

Dans le cas 3) :

- générer un échantillon $\xi'^{(k)}$ à partir de la loi a posteriori $p(\xi'|Y,\gamma_b^{(k-1)},T^{(k-1)},K'^{(k-1)})$ conformément à l'algorithme A,
- générer un échantillon $\gamma_b^{(k)}$ à partir de la loi a posteriori $p(\gamma_b|Y,\xi'^{(k)},T^{(k-1)},K'^{(k-1)})$ conformément à l'algorithme B,

- générer un échantillon $T^{(k)}$ à partir de la loi a posteriori $p(T|Y,\gamma_b^{(k)},\xi'^{(k)},K'^{(k-1)})$ conformément à l'algorithme C,
- générer un échantillon $K^{(k)}$ à partir de la loi a posteriori $p(K'Y,\gamma_b^{(k)},\xi'^{(k)},T^{(k)},x^{(k-1)})$ conformément à l'algorithme A, et, du fait de la structure hiérarchique du modèle tel qu'illustré par la figure 2 :
- générer un échantillon $x^{(k)}$ à partir de la loi a posteriori $p(x|K'^{(k)},B^{(k-1)})$ conformément à l'algorithme A,
- générer un échantillon $B^{(k)}$ à partir de la loi a posteriori $Pr(B|x^{(k)})$ conformément à l'algorithme D,

Le processeur 28 exécute ensuite une étape d'estimation 108 lors de laquelle l'estimateur maximum a posteriori de la variable à valeurs discrètes B est calculé, pour l'obtention d'une estimation $\hat{B}$, et l'estimateur espérance a posteriori conditionnelle des variables à valeurs continues $\gamma_b$, $\xi$, T, K, K*, $\alpha$, x, est calculé pour l'obtention d'estimations $\hat{\gamma_b},\hat{\xi},\hat{T},\hat{K},\hat{K^*},\hat{\alpha},\hat{x}$ (selon le cas considéré) en ne retenant que les échantillons k tels que $B^{(k)} = \hat{B}$. En pratique, l'estimateur maximum a posteriori de la variable aléatoire B est approché par la sélection de l'état qui apparaît le plus de fois entre les indices kmin et kmax. En pratique également, l'estimateur espérance a posteriori d'une variable aléatoire est approché par la moyenne de ses échantillons pris entre un indice kmin et l'indice kmax de fin de boucle en n'utilisant que les échantillons tels que $B^{(k)} = \hat{B}$, kmin étant une valeur de « temps de chauffe » prédéterminée jugée nécessaire pour que, lors de l'échantillonnage de Gibbs, la loi de tirage aléatoire converge vers la loi a posteriori jointe, cette dernière pouvant être également appelée loi cible. Par exemple, pour une boucle de kmax=500 échantillons, une valeur de temps de chauffe de kmin=200 (c'est-à-dire 40% du nombre total d'itérations) semble raisonnable.

Enfin, lors d'une dernière étape 110, les estimations $\hat{\gamma_b},\hat{\xi},\hat{T},\hat{K},\hat{K^*},\hat{\alpha},\hat{x}$ et $\hat{B}$ sont retournées, éventuellement accompagnées d'un facteur d'incertitude. En particulier, l'estimation $\hat{x}$ donne un ensemble de valeurs pour les concentrations de protéines d'intérêt dans l'échantillon E et l'estimation $\hat{B}$ constitue une aide au diagnostic. On notera que ce diagnostic peut ensuite être rendu dans un second temps par un praticien sur la base de cette estimation mais ne fait pas partie de l'objet de l'invention. On notera également qu'il convient de donner seulement des probabilités empiriques pour chaque état biologique B que l'on peut exprimer sous la forme $\dfrac{n_B}{k\max - k\min + 1}$ où $n_B$ est le nombre de fois où un événement B a été sélectionné entre les itérations kmin et kmax.

De nombreuses méthodes de calcul de facteurs d'incertitudes sont connues et peuvent être appliquées. Elles ne seront pas détaillées ici, mais elles peuvent notamment être basées sur l'étude d'histogrammes des échantillons obtenus à l'étape 106.

La première phase 200 de calibrage externe de la chaîne de traitement biologique 12 comporte une première étape de mesure 202 lors de laquelle, conformément au montage de la figure 1, l'échantillon de protéines de calibrage externe $E_{CALIB1}$ traverse toute la chaîne de traitement 12 du dispositif 10 pour la fourniture d'un chromatospectrogramme $Y_{CALIB1}$.

Le traitement appliqué par le processeur 28 au signal $Y_{CAUB1}$ consiste à déterminer des valeurs non encore connues de paramètres certains, c'est-à-dire des paramètres qui restent en réalité relativement constants d'un traitement biologique à l'autre. Ces paramètres certains sont alors considérés et modélisés par des constantes dans la chaîne de traitement biologique 12. Il s'agit par exemple des coefficients de la matrice de digestion D ou de la matrice de correction des gains de digestion $\alpha$ si D est connue a priori, de la largeur des pics chromatographiques et spectrométriques et des proportions de protéines d'intérêt à neutrons ou charges supplémentaires. Ce traitement peut consister également à déterminer des paramètres stables (par exemple moyenne ou variance) des modèles de probabilités a priori de paramètres incertains. Ces paramètres stables sont alors également considérés et modélisés par des constantes.

Dans l'échantillon de protéines de calibrage externe, certains des paramètres incertains sont cette fois-ci connus comme le vecteur des concentrations en protéines, mais comme pour la phase principale 100, la détermination se fait par application d'un échantillonnage numérique conforme au procédé de Monte-Carlo à chaîne de Markov. Elle se fait cette fois-ci cependant de façon classique sur les paramètres certains non connus et est illustrée par la référence 204. L'étape 204 reproduit donc une partie des étapes de détermination 104, 106 et 108 de la phase principale 100.

Enfin, lors d'une dernière étape 206 de la première phase de calibrage externe, les paramètres certains et stables déterminés à l'étape précédente sont enregistrés dans la base de données 32.

La phase de sélection 300 suppose que tous les paramètres certains et stables sont connus. Elle comporte une première étape de mesure 302 lors de laquelle, conformément au montage de la figure 1, les échantillons $E_{REF}$ traversent chacun toute la chaîne de traitement 12 du dispositif 10 pour la fourniture de chromatospectrogrammes $Y_{REF}$. Dans le cas où les états biologiques possibles sont un état sain S et un état pathologique P, l'ensemble des échantillons $E_{REF}$ comporte un sous-ensemble d'échantillons connus comme étant sains et un sous-ensemble d'échantillons connus comme étant pathologiques. L'objectif de la phase de sélection 300 est de sélectionner, parmi un ensemble de protéines candidates, les protéines dont les concentrations sont les plus discriminantes par rapport à ces deux états biologiques. On passe alors par une étape d'estimation de ces concentrations, sauf si l'on peut se passer de cette étape pour accéder directement aux protéines d'intérêt. Dans ce cas cependant, le marquage isotopique ne peut se faire, suite à quoi les concentrations seront seulement connues de façon relative. C'est la raison pour laquelle le paramètre de gain $\xi$ et le coefficient de digestion $\alpha$ ne sont pas des variables d'intérêt dans cette phase et pour laquelle également l'échantillon E* n'est pas intégré à chaque échantillon de l'ensemble des échantillons $E_{REF}$. Dans ce cas, on attribue arbitrairement une valeur à $\xi$.

Comme pour la phase principale 100, la détermination se fait par un échantillonnage numérique conforme au procédé de Monte-Carlo à chaîne de Markov, sachant que cette fois-ci l'état biologique B n'est pas une variable aléatoire, mais une constante connue pour chacun des sous-ensembles précités.

Ainsi pour chaque échantillon de l'ensemble $E_{REF}$, lors d'une étape d'initialisation 304, les variables aléatoires $\gamma_b$, T, K et x sont chacune initialisées par le processeur 28 à une première valeur $\gamma_b^{(0)}$, $T^{(0)}$, $K^{(0)}$ et $x^{(0)}$. Dans cette phase, x est le vecteur des concentrations des protéines candidates. Si une valeur pour le coefficient de digestion est connue, elle pourra être mise à profit dans le modèle.

Le processeur 28 exécute alors une boucle 306 d'échantillonnage de Gibbs de chacune des variables aléatoires initialisées au vu de leurs lois de probabilité a posteriori conditionnelles respectives. Plus précisément, pour k variant de 1 à kmax, la boucle 306 comporte les échantillonnages successifs suivants :

- générer un échantillon $\gamma_b^{(k)}$ à partir de la loi a posteriori $p(\gamma_b|Y, T^{(k-1)}, K^{(k-1)})$ conformément à l'algorithme B,
- générer un échantillon $T^{(k)}$ à partir de la loi a posteriori $p(T|Y, \gamma_b^{(k)}, K^{(k-1)})$ conformément à l'algorithme C,
- générer un échantillon $K^{(k)}$ à partir de la loi a posteriori $p(K|\gamma_b^{(k)}, T^{(k)}, \alpha^{(k-1)}, x^{(k-1)})$ conformément à l'algorithme A,
- générer un échantillon $x^{(k)}$ à partir de la loi a posteriori $p(x|K^{(k)})$ conformément à l'algorithme A.

Le processeur 28 exécute ensuite une étape d'estimation 308 lors de laquelle l'estimateur espérance a posteriori est calculé notamment pour la variable x pour l'obtention d'une estimation $\hat{x}$. En pratique, l'estimateur espérance a posteriori est approché par la moyenne des échantillons pris entre les indices kmin et kmax. Les étapes 304 à 308 étant exécutées pour chaque échantillon de référence sain ou pathologique, on obtient finalement pour chaque état biologique S ou P un ensemble de valeurs de $\hat{x}$. On observe que la succession d'étapes 304, 306, 308 reproduit partiellement les étapes de détermination 104, 106, 108 de la phase principale 100.

En supposant que $p(x|B=S)$ et $p(x|B=P)$ suivent des lois normales N de moyennes respectives $\mu_S$, $\mu_P$ et d'inverses-matrices de covariance $\Gamma_S$, $\Gamma_P$, on obtient un modèle relatif de ces lois au cours d'une étape 310 en estimant de façon connue en soi une approximation des valeurs vectorielles ou matricielles $\mu_S$, $\mu_P$, $\Gamma_S$ et $\Gamma_P$, à partir de l'ensemble de valeurs de $\hat{x}$. En particulier, pour la p-ième protéine candidate, on obtient des valeurs scalaires de moyennes et d'écarts types $\mu^p_S$, $\mu^p_P$, $\sigma^p_S$ et $\sigma^p_P$ pour les lois de probabilités que suivent $p(x_p|B = S)$ et $p(x_p|B = P)$, où $x_p$ désigne la concentration en p-ième protéine. Ces lois sont notées respectivement $N\left(t, \mu^p_S, \sigma^p_S\right)$ et $N\left(t, \mu^p_P, \sigma^p_P\right)$ pour la p-ième protéine.

Sur la base de ces lois, au cours d'une étape 312 et pour chaque protéine candidate en supposant en outre que les protéines sont indépendantes les unes des autres, le processeur 28 détermine la valeur $x^p_0$ de concentration $x_p$ pour laquelle l'erreur de première espèce relativement à l'état biologique B est égale à l'erreur de seconde espèce.

En notant $$\varphi^p_S(x) = \int_{-\infty}^x N\left(t, \mu^p_S, \sigma^p_S\right) dt = p\left(x_p \leq x | B = S\right)$$ et

$$\varphi^p_P(x) = \int_{-\infty}^x N\left(t, \mu^p_P, \sigma^p_P\right) dt = p\left(x_p \leq x | B = P\right),$$ cela revient à déterminer la valeur $x^p_0$ de $x_p$ pour laquelle

$$\varphi^p_S\left(x^p_0\right) = 1 - \varphi^p_P\left(x^p_0\right).$$ Un score $S_p$ est alors attribué à la p-ième protéine sur la base de la valeur de $\varphi^p_S\left(x^p_0\right)$

ou $\varphi^p_P\left(x^p_0\right)$ selon que la protéine considérée est sur ou sous exprimée dans l'état P. En d'autres termes, on retient des deux valeurs $\varphi^p_S\left(x^p_0\right)$ ou $\varphi^p_P\left(x^p_0\right)$ celle qui est la plus élevée en $x^p_0$. Le score Sp peut alors être exprimé selon la formule suivante : $$S_p = 2 \cdot Max\left(\varphi^p_S\left(x^p_0\right), \varphi^p_P\left(x^p_0\right)\right) - 1.$$

Sachant que les fonctions $\varphi^p_S$ et $\varphi^p_P$ sont positives, croissantes et à valeurs comprises entre 0 (en $-\infty$) et

1 (en +∞), Sp est à valeur dans [0,1]. Puisqu'en outre $\varphi_S^p\left(x_0^p\right)=1-\varphi_P^p\left(x_0^p\right)$, alors plus Sp est proche de 1, plus

cela signifie que $\varphi_S^p\left(x_0^p\right)$ et $\varphi_P^p\left(x_0^p\right)$ sont différents et que la p-ième protéine est discriminante en termes de concentration relativement à l'état biologique B.

Au vu de ce qui précède, lors d'une dernière étape 314 de la phase de sélection 300, le processeur 28 sélectionne des protéines d'intérêt parmi les protéines candidates sur la base des scores Sp calculés précédemment. Par exemple, seules sont retenues les protéines dont le score est supérieur à un seuil S prédéterminé ou seules sont retenues les P protéines (P = 3, 5 ou autre) dont les scores sont les plus élevés. Lors de cette étape également, les protéines d'intérêt sélectionnées et identifiées sont enregistrées dans la base de données 32.

Les étapes 312 et 314 de sélection de P protéines d'intérêt ont été détaillées sur la base d'une hypothèse d'indépendance des protéines candidates. Mais elles peuvent être généralisées dans une approche de sélection globale de sous-protéome différentiant en cas de dépendance des protéines, par exemple de la façon suivante.

A partir d'une liste de protéines candidates, on calcule le centre de chaque nuage de points (i.e. valeurs de $\hat{x}$ pour S ou P) obtenu à l'issue de l'étape 308. En notant V le vecteur qui lie ces deux centres, on projette les points de l'espace multidimensionnel sur le sous-espace monodimensionnel engendré par V, la projection d'une gaussienne restant une gaussienne. En utilisant ce qui précède, on peut alors calculer le score de différentiation d'un ensemble de protéines. En éliminant au fur et à mesure certaines d'entre elles, on finit par se retrouver avec une sélection de protéines d'intérêt.

De même, l'homme du métier saura également généraliser la sélection des protéines d'intérêt à un état biologique ou chimique B comportant plus de deux valeurs.

La seconde phase optionnelle 400 de calibrage externe de la chaîne de traitement biologique 12 est identique à la première phase 200 de calibrage externe, si ce n'est que l'échantillon de protéines de calibrage externe $E_{CALIB1}$ utilisé en phase 200 est remplacé par au moins un échantillon de protéines de calibrage externe $E_{CALIB2}$ dans lequel les protéines sont choisies parmi les protéines d'intérêt sélectionnées en phase 300. Des échantillons de marquage peuvent être utilisés.

Les étapes 402, 404 et 406 de cette seconde phase optionnelle 400 de calibrage externe sont identiques aux étapes 202, 204 et 206 de sorte qu'elles ne seront pas de nouveau décrites. Des coefficients comme la matrice $\alpha$ des rendements de digestion pourront cependant être négligés dans les étapes 200 mais calibrés dans les étapes 400 du fait de l'utilisation d'un nombre restreint de protéines et d'un modèle de chaîne d'acquisition plus précis.

Ainsi, les paramètres certains et stables dont la détermination est affinée par l'exécution de cette phase optionnelle 400 sont mis à jour dans la base de données 32.

La phase d'apprentissage 500 suppose que tous les paramètres certains et stables sont connus (phase 200 et éventuellement phase 400) et que les protéines d'intérêt sont sélectionnées et identifiées (phase 300). Elle comporte une première étape de mesure 502 lors de laquelle, conformément au montage de la figure 1, les échantillons $E_{REF}$ traversent chacun toute la chaîne de traitement 12 du dispositif 10 pour la fourniture de chromatospectrogrammes Y. L'échantillon de protéines de marquage E* est intégré à chaque échantillon de l'ensemble des échantillons $E_{REF}$ parce que, dans cette phase d'apprentissage, il est nécessaire d'estimer les concentrations de protéines d'intérêt de façon absolue et donc de connaître le paramètre de gain $\xi$. Comme précédemment, dans le cas où les états biologiques possibles sont un état sain S et un état pathologique P, l'ensemble des échantillons $E_{REF}$ comporte un sous-ensemble d'échantillons connus comme étant sains et un sous-ensemble d'échantillons connus comme étant pathologiques.

Comme pour la phase principale 100, la détermination des concentrations de protéines d'intérêt se fait par un échantillonnage numérique conforme au procédé de Monte-Carlo à chaîne de Markov, sachant que cette fois-ci encore l'état biologique B n'est pas une variable aléatoire, mais une constante connue pour chacun des sous-ensembles précités.

Ainsi pour chaque échantillon de l'ensemble $E_{REF}$, lors d'une étape d'initialisation 504, les variables aléatoires $\xi$, $\gamma_b$, T, K, K*, $\alpha$ et x sont chacune initialisées par le processeur 28 à une première valeur $\xi^{(0)}$, $\gamma_b^{(0)}$, $T^{(0)}$, $K^{(0)}$, $K^{*(0)}$, $\alpha^{(0)}$ et $x^{(0)}$. Dans cette phase, x est le vecteur des concentrations des protéines d'intérêt.

Le processeur 28 exécute alors une boucle 506 d'échantillonnage de Gibbs de chacune des variables aléatoires initialisées au vu de leurs lois de probabilité a posteriori conditionnelles respectives. Plus précisément, pour k variant de 1 à kmax, la boucle 506 comporte les échantillonnages successifs suivants :

Dans le cas 1)

- générer un échantillon $\gamma_b^{(k)}$ à partir de la loi a posteriori $p(\gamma_b|Y,\xi^{(k)},T^{(k-1)},K^{(k-1)},K^{*(k-1)})$ conformément à l'algorithme B,
- générer un échantillon $T^{(k)}$ à partir de la loi a posteriori $p(T|Y,\gamma_b^{(k)},\xi^{(k)},K^{(k-1)},K^{*(k-1)})$ conformément à l'algorithme C,

- générer un échantillon $K^{(k)}$ à partir de la loi a posteriori $p(K|Y,\gamma_b^{(k)},\xi^{(k)},T^{(k)},K^{*(k-1)},\alpha^{(k-1)},x^{(k-1)})$ conformément à l'algorithme A,
- générer un échantillon $K^{*(k)}$ à partir de la loi a posteriori $p(K^*|Y,\gamma_b^{(k)},\xi^{(k)},T^{(k)},K^{(k)},\alpha^{(k-1)},x^{(k-1)})$ conformément à l'algorithme A,
- générer un échantillon $\alpha^{(k)}$ à partir de la loi a posteriori $p(\alpha|K^{(k)},K^{*(k)},x^{(k-1)})$ conformément à l'algorithme E,
- générer un échantillon $x^{(k)}$ à partir de la loi a posteriori $p(x|K^{(k)},\alpha^{(k)})$ conformément à l'algorithme A,

Dans le cas 2)

- générer un échantillon $\xi^{(k)}$ à partir de la loi a posteriori $p(\xi|Y,\gamma_b^{(k-1)},T^{(k-1)},K^{(k-1)})$ conformément à l'algorithme A,
- générer un échantillon $\gamma_b^{(k)}$ à partir de la loi a posteriori $p(\gamma_b|Y,\xi^{(k)},T^{(k-1)},K^{(k-1)})$ conformément à l'algorithme B,
- générer un échantillon $T^{(k)}$ à partir de la loi a posteriori $p(T|Y,\gamma_b^{(k)},\xi^{(k)},K^{(k-1)})$ conformément à l'algorithme C,
- générer un échantillon $K^{(k)}$ à partir de la loi a posteriori $p(K|Y,\gamma_b^{(k)},\xi^{(k)},T^{(k)},x^{(k-1)})$ conformément à l'algorithme A,
- générer un échantillon $x^{(k)}$ à partir de la loi a posteriori $p(x|K^{(k)})$
  conformément à l'algorithme A,

Dans le cas 3)

- générer un échantillon $\xi'^{(k)}$ à partir de la loi a posteriori $p(\xi,|Y,\gamma_b^{(k-1)},T^{(k-1)},K'^{(k-1)})$ conformément à l'algorithme A,
- générer un échantillon $\gamma_b^{(k)}$ à partir de la loi a posteriori $p(\gamma_b|Y,\xi'^{(k)},T^{(k-1)},K'^{(k-1)})$ conformément à l'algorithme B,
- générer un échantillon $T^{(k)}$ à partir de la loi a posteriori $p(T|Y,\gamma_b^{(k)},\xi'^{(k)},K'^{(k-1)})$ conformément à l'algorithme C,
- générer un échantillon $K^{(k)}$ à partir de la loi a posteriori $p(K'|Y,\gamma_b^{(k)},\xi'^{(k)},T^{(k)},x^{(k-1)})$ conformément à l'algorithme A,
- générer un échantillon $x^{(k)}$ à partir de la loi a posteriori $p(x|K'^{(k)})$ conformément à l'algorithme A,

Le processeur 28 exécute ensuite une étape d'estimation 508 lors de laquelle l'estimateur espérance a posteriori est calculé notamment pour la variable x pour l'obtention d'une estimation $\hat{x}$. En pratique, l'estimateur espérance a posteriori est approché par la moyenne des échantillons pris entre les indices kmin et kmax. Les étapes 504 à 508 étant exécutées pour chaque échantillon de référence sain ou pathologique, on obtient finalement pour chaque état biologique S ou P un ensemble de valeurs de $\hat{x}$. On observe que la succession d'étapes 504, 506, 508 reproduit partiellement les étapes de détermination 104, 106, 108 de la phase principale 100.

En supposant de nouveau que $p(x|B=S)$ et $p(x|B=p)$ suivent des lois normales N de moyennes respectives $\mu_S$, $\mu_P$ et d'inverses-matrices de covariance $\Gamma_S$, $\Gamma_P$, on obtient un modèle absolu de ces lois au cours d'une étape 510 en estimant de façon connue en soi une approximation des valeurs vectorielles ou matricielles $\mu_S$, $\mu_P$, $\Gamma_S$ et $\Gamma_P$, à partir de l'ensemble de valeurs de $\hat{x}$. Lors de cette étape également, les paramètres précités $\mu_S$, $\mu_P$, $\Gamma_S$ et $\Gamma_P$ sont enregistrées dans la base de données 32.

Lors des étapes 508 et 510, il est possible également, si cela n'est pas déjà connu, d'estimer de la même façon mais cette fois-ci indépendamment de l'état biologique B, les paramètres des lois de probabilité a priori pour les paramètres de gain $\xi$, de bruit $\gamma_b$, ou de temps de rétention T.

Il apparaît clairement qu'un procédé tel que celui décrit précédemment, mis en oeuvre par le dispositif d'estimation 10, permet, grâce à une modélisation hiérarchique fine de la chaîne de traitement 12, de fournir une estimation fiable de paramètres biologiques ou chimiques (par exemple les concentrations) de composants d'intérêts prédéterminés, et même éventuellement de constituer une aide au diagnostic lorsqu'une phase d'apprentissage 500 telle que celle décrite précédemment peut être menée. En particulier, ce procédé excelle à évaluer correctement des pics de mesure en présence de bruit important ou superposés à d'autres pics d'un chromatospectrogramme, ce que les méthodes classiques d'analyse de pics ou d'analyses spectrales réalisent moins bien.

Des applications concrètes de ce procédé comportent notamment la détection de marqueurs cancéreux (dans ce cas les composants d'intérêt sont des protéines) dans un échantillon biologique de sang ou d'urine.

On notera par ailleurs que l'invention n'est pas limitée au mode de réalisation décrit précédemment. Il apparaîtra en effet à l'homme de l'art que diverses modifications peuvent être apportées au mode de réalisation décrit ci-dessus, à la lumière de l'enseignement qui vient de lui être divulgué.

Notamment, l'état biologique B peut prendre plus de deux valeurs discrètes pour la détection d'une pathologie parmi plusieurs possibles ou pour se réserver la possibilité de diagnostiquer un état biologique incertain.

Notamment également, les composants d'intérêt ne sont pas nécessairement des protéines, mais peuvent être plus généralement des molécules ou des assemblages moléculaires pour une analyse biologique ou chimique.

Plus généralement, dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

**Revendications**

1. Procédé d'estimation de paramètres biologiques ou chimiques (x, B) dans un échantillon (E), comportant les étapes suivantes :

   - faire passer (102) l'échantillon (E) par une chaîne de traitement (12),
   - obtenir ainsi un signal (Y) représentatif des paramètres biologiques ou chimiques (x, B) en fonction d'au moins une variable de la chaîne de traitement, et
   - estimer (104, 106, 108, 110) les paramètres biologiques ou chimiques (x, B) à l'aide d'un dispositif de traitement de signal (14) par inférence bayésienne, sur la base d'une modélisation analytique directe dudit signal (Y) en fonction des paramètres biologiques ou chimiques (x, B) et de paramètres techniques ($\gamma_b$, $\xi$, T, K, K*, $\alpha$) de la chaîne de traitement (12),

   **caractérisé en ce qu'**au moins deux desdits paramètres biologiques ou chimiques (x, B) ou techniques ($\gamma_b$, $\xi$, T, K, K*, $\alpha$) en fonction desquels la modélisation analytique directe dudit signal (Y) est définie ont une relation de dépendance probabiliste entre eux, et **en ce que** le traitement de signal par inférence bayésienne est en outre réalisé sur la base d'une modélisation par loi de probabilité a priori conditionnelle de cette dépendance.

2. Procédé d'estimation de paramètres biologiques ou chimiques (x, B) selon la revendication 1, dans lequel l'étape (104, 106, 108, 110) d'estimation des paramètres biologiques ou chimiques (x, B) comporte, par approximation de la loi de probabilité a posteriori jointe des paramètres biologiques ou chimiques (x, B) et des paramètres techniques ($\gamma_b$, $\xi$, T, K, K*, $\alpha$) conditionnellement au signal obtenu (Y) à l'aide d'un algorithme d'échantillonnage stochastique :

   - une boucle (106) d'échantillonnage d'au moins une partie (x, B) des paramètres biologiques ou chimiques de l'échantillon (E) et d'au moins une partie ($\gamma_b$, $\xi$, T, K, K*, $\alpha$) des paramètres techniques de la chaîne de traitement, fournissant des valeurs échantillonnées de ces paramètres, et
   - une estimation (108) de ladite au moins une partie des paramètres biologiques ou chimiques et techniques (x, B, $\gamma_b$, $\xi$, T, K, K*, $\alpha$) calculée à partir des valeurs échantillonnées fournies.

3. Procédé d'estimation de paramètres biologiques ou chimiques (x, B) selon la revendication 2, dans lequel l'estimation (108) de ladite au moins une partie des paramètres biologiques ou chimiques et techniques (x, B, $\gamma_b$, $\xi$, T, K, $\alpha$) calculée à partir des valeurs échantillonnées fournies comporte :

   - un calcul de l'estimateur espérance ou médiane ou maximum a posteriori pour chaque paramètre à valeurs continues (x, $\gamma_b$, $\xi$, T, K, K*, $\alpha$),
   - un calcul de l'estimateur maximum a posteriori pour chaque paramètre à valeurs discrètes (B), ou
   - un calcul de probabilité d'au moins une partie des paramètres biologiques ou chimiques et techniques (x, B, $\gamma_b$, $\xi$, T, K, K*, $\alpha$).

4. Procédé d'estimation de paramètres biologiques ou chimiques (x, B) selon l'une quelconque des revendications 1 à 3, dans lequel les paramètres biologiques ou chimiques comportent un vecteur représentatif de concentrations de composants de l'échantillon, le procédé comportant en outre une phase préalable de calibrage (200), dit calibrage externe, comportant les étapes suivantes :

   - faire passer (202) un échantillon ($E_{CALIB1}$) de composants de calibrage externe par la chaîne de traitement (12), ces composants de calibrage externe étant choisis parmi les composants de l'échantillon et étant de concentration connue,
   - obtenir ainsi un signal représentatif des concentrations des composants de calibrage externe en fonction d'au moins une variable de la chaîne de traitement (12) et d'au moins un paramètre constant de valeur inconnue et/ou d'au moins un paramètre statistique stable de la chaîne de traitement,
   - appliquer (204) au moins une partie de l'étape d'estimation des paramètres biologiques ou chimiques à l'aide du dispositif de traitement de signal (14) par inférence bayésienne, pour en déduire la valeur de chaque paramètre constant de valeur inconnue et/ou de chaque paramètre statistique stable de la chaîne de traitement (12),
   - enregistrer (206) en mémoire (32) chaque valeur de paramètre constant et/ou chaque valeur de paramètre statistique stable déduite précédemment.

5. Procédé d'estimation de paramètres biologiques ou chimiques (x, B) selon l'une quelconque des revendications 1 à 4, dans lequel les paramètres biologiques ou chimiques (x, B) sont relatifs à des protéines et l'échantillon (E) comporte l'un des éléments de l'ensemble constitué de sang, plasma et urine.

6. Procédé d'estimation de paramètres biologiques ou chimiques (x, B) selon l'une quelconque des revendications 1 à 5, dans lequel :

   - le signal (Y) représentatif des paramètres biologiques ou chimiques (x, B) est exprimé en fonction de concentrations moléculaires d'espèces (K),
   - ces espèces (K) sont issues d'une décomposition d'espèces moléculaires d'intérêt (x),
   - le procédé comporte une estimation d'une quantité de ces espèces obtenue suite à la décomposition des espèces moléculaires d'intérêt (x).

7. Procédé d'estimation de paramètres biologiques ou chimiques (x, B) selon la revendication 6, dans lequel :

   - les espèces comportent des peptides ou des polypeptides,
   - les espèces moléculaires d'intérêt comportent des protéines contenant chacune une quantité de ces peptides ou polypeptides,
   - on définit un rendement de digestion ($\alpha$) des protéines sous la forme d'une matrice de coefficients $\alpha_{ip}$, où $\alpha_{ip}$ désigne le rendement de digestion de la p-ième protéine par rapport au i-ième peptide ou polypeptide, de sorte que les concentrations moléculaires (K) de peptides ou polypeptides soient liées à un vecteur (x) représentatif de concentrations des protéines via une matrice de digestion (D) et ce rendement de digestion ($\alpha$),
   - le procédé comporte une estimation de ce rendement de digestion ($\alpha$).

8. Procédé d'estimation de paramètres biologiques ou chimiques (x, B) selon la revendication 6, dans lequel :

   - les espèces comportent des peptides ou des polypeptides,
   - les espèces moléculaires d'intérêt comportent des protéines contenant une quantité de ces peptides ou polypeptides,
   - on définit un gain global ($\xi$) de la chaîne de traitement (12) de manière à modéliser le signal (Y) représentatif des paramètres biologiques ou chimiques (x, B) par la relation $Y = \xi K$, où K est un vecteur représentatif de concentrations des peptides ou polypeptides,
   - le procédé comporte une estimation de ce gain global ($\xi$).

9. Procédé d'aide au diagnostic comportant les étapes d'un procédé d'estimation de paramètres biologiques ou chimiques (x, B) selon l'une quelconque des revendications 1 à 8, dans lequel les paramètres biologiques ou chimiques (x, B) de l'échantillon (E) comportent un paramètre d'état biologique ou chimique (B) à valeurs discrètes, chaque valeur discrète possible de ce paramètre étant associée à un état possible de l'échantillon (E), et un vecteur représentatif de concentrations de composants de l'échantillon (E), et dans lequel le vecteur représentatif des concentrations (x) et le paramètre d'état biologique ou chimique (B) ayant une relation de dépendance probabiliste entre eux, le traitement de signal par inférence bayésienne est en outre réalisé sur la base d'une modélisation par loi de probabilité a priori du vecteur représentatif des concentrations (x) conditionnellement aux valeurs possibles du paramètre d'état biologique ou chimique (B).

10. Procédé d'aide au diagnostic selon la revendication 9, comportant une phase préalable d'apprentissage (500) comportant les étapes suivantes :

   - faire passer (502) successivement une pluralité d'échantillons de référence ($E_{REF}$) par la chaîne de traitement (12), la valeur du paramètre d'état biologique ou chimique (B) de chaque échantillon de référence étant connue,
   - obtenir ainsi un signal représentatif des concentrations (x) des composants pour chaque échantillon de référence ($E_{REF}$) en fonction d'au moins une variable de la chaîne de traitement (12),
   - appliquer (504, 506, 508) au moins une partie de l'étape d'estimation des paramètres biologiques ou chimiques à l'aide du dispositif de traitement de signal par inférence bayésienne, pour en déduire des valeurs des concentrations des composants pour chaque échantillon de référence ($E_{REF}$),
   - en déduire (510) des paramètres de loi de probabilité a priori du vecteur représentatif des concentrations (x) conditionnellement aux valeurs possibles du paramètre d'état biologique ou chimique (B), et

- enregistrer (510) en mémoire (32) ces paramètres de loi de probabilité.

11. Procédé d'aide au diagnostic selon la revendication 9 ou 10, comportant une phase préalable (300) de sélection desdits composants parmi un ensemble de composants candidats, cette phase préalable de sélection (300) comportant les étapes suivantes :

- faire passer (302) successivement une pluralité d'échantillons de référence ($E_{REF}$) par la chaîne de traitement (12), la valeur du paramètre d'état biologique ou chimique (B) de chaque échantillon de référence étant connue,
- obtenir ainsi un signal représentatif des concentrations (x) des composants candidats pour chaque échantillon de référence ($E_{REF}$) en fonction d'au moins une variable de la chaîne de traitement (12),
- appliquer (304, 306, 308) au moins une partie de l'étape d'estimation des paramètres biologiques ou chimiques à l'aide du dispositif de traitement de signal par inférence bayésienne, pour en déduire des valeurs représentatives des concentrations (x) des composants candidats pour chaque échantillon de référence ($E_{REF}$),
- en déduire (310) des paramètres de distributions du vecteur représentatif des concentrations (x) des composants candidats pour chaque valeur discrète du paramètre d'état biologique ou chimique (B),
- sélectionner (312, 314), parmi les composants candidats, ceux dont les distributions sont les plus dissemblables les unes des autres en fonction des valeurs du paramètre d'état biologique ou chimique (B).

12. Dispositif (10) d'estimation de paramètres biologiques ou chimiques (x, B) dans un échantillon (E), comportant :

- une chaîne (12) de traitement de l'échantillon (E) conçue pour la fourniture d'un signal (Y) représentatif des paramètres biologiques ou chimiques (x, B) en fonction d'au moins une variable de la chaîne de traitement,
- un dispositif de traitement de signal (14) conçu pour appliquer, en combinaison avec la chaîne de traitement (12), un procédé (100) d'estimation de paramètres biologiques ou chimiques (x, B) ou d'aide au diagnostic selon l'une quelconque des revendications 1 à 11.

13. Dispositif (10) d'estimation de paramètres biologiques ou chimiques (x, B) selon la revendication 12, dans lequel la chaîne de traitement (12) comporte une colonne de chromatographie (22) et/ou un spectromètre de masse (26) et est conçue pour la fourniture d'un signal (Y) représentatif des concentrations (x) de composants de l'échantillon (E) en fonction d'un temps de rétention (T) dans la colonne de chromatographie (22) et/ou d'un rapport masse/charge dans le spectromètre de masse (26).

**Patentansprüche**

1. Verfahren zum Schätzen von biologischen oder chemischen Parametern (x, B) in einer Probe (E), das die folgenden Schritte umfasst:

- die Probe (E) durch eine Behandlungskette (12) durchlaufen lassen (102),
- auf diese Weise ein Signal (Y) erhalten, das für die biologischen oder chemischen Parameter (x, B), abhängig von mindestens einer Variablen der Behandlungskette, repräsentativ ist, und
- schätzen (104, 106, 108, 110) der biologischen oder chemischen Parameter (x, B) mithilfe einer Vorrichtung zur Signalverarbeitung (14) durch Bayessche Inferenz, auf der Grundlage einer direkten analytischen Modellierung des genannten Signals (Y) in Abhängigkeit der biologischen oder chemischen Parameter (x, B) und der technischen Parameter ($\gamma_b$, $\xi$, T, K, K*, $\alpha$) der Behandlungskette (12),

**dadurch gekennzeichnet, dass** mindestens zwei der genannten biologischen oder chemischen (x, B) oder der technischen ($\gamma_b$, $\xi$, T, K, K*, $\alpha$) Parameter, nach denen die direkte analytische Modellierung des genannten Signals (Y) definiert wird, unter ihnen ein auf Wahrscheinlichkeiten beruhender Abhängigkeitszusammenhang aufweisen, und dadurch, dass die Signalverarbeitung durch Bayessche Inferenz des Weiteren aufgrund einer Wahrscheinlichkeits-Modellierung dieser Abhängigkeit realisiert wird, die a priori bedingt ist.

2. Verfahren zum Schätzen von biologischen oder chemischen Parametern (x, B) nach Anspruch 1, in dem der Schritt der Schätzung (104, 106, 108, 110) der biologischen oder chemischen Parameter (x, B) durch die Näherung der a posteriori Wahrscheinlichkeitsgesetzmäßigkeit mit Bezug auf biologischen oder chemischen Parameter (x, B) und der technischen Parameter ($\gamma_b$, $\xi$, T, K, K*, $\alpha$) bedingt durch das mithilfe eines Algorithmus' zur stochastischen Probennahme erhaltene Signal (Y) Folgendes umfasst:

- eine Probenschleife (106) von mindestens einem Teil (x, B) der biologischen oder chemischen Parameter der Probe (E) und von mindestens einem Teil ($\gamma_b$, $\xi$, T, K, K*, $\alpha$) der technischen Parameter der Behandlungskette, die Probewerte dieser Parameter bereitstellt, und

- eine Schätzung (108) des mindestens einen genannten Teils der biologischen oder chemischen und technischen Parameter (x, B, $\gamma_b$, $\xi$, T, K, K*, $\alpha$), die ausgehend von den bereitgestellten Probewerten berechnet wird.

3. Verfahren zum Schätzen von biologischen oder chemischen Parametern (x, B) nach Anspruch 2, in dem die Schätzung (108) des mindestens einen genannten Teils der biologischen oder chemischen und technischen Parameter (x, B, $\gamma_b$, $\xi$, T, K, K*, $\alpha$), die ausgehend von den bereitgestellten Probewerten berechnet wird, Folgendes umfasst:

- eine Berechnung des Schätzers für den Erwartungswert oder für den Median oder Maximalwert a posteriori für jeden Parameter mit kontinuierlichen Werten (X, $\gamma_b$, $\xi$, T, K, K*, $\alpha$),
- eine Berechnung des Schätzers für den Maximalwert a posteriori für jeden Parameter mit diskreten Werten (B), oder
- eine Wahrscheinlichkeitsrechnung für mindestens einen Teil der biologischen oder chemischen und technischen Parameter (x, B, $\gamma_b$, $\xi$, T, K, K*, $\alpha$).

4. Verfahren zum Schätzen von biologischen oder chemischen Parametern (x, B) nach einem der Ansprüche von 1 bis 3, in dem die biologischen oder chemischen Parameter einen Vektor umfassen, der die Konzentrationen der Bestandteile der Probe wiedergibt, wobei das Verfahren des Weiteren eine vorgelagerte Kalibrier-Phase (200), genannt externes Kalibrieren, umfasst, die folgende Schritte umfasst:

- durchlaufen lassen (202) einer Probe ($E_{CALIB1}$) von externen Kalibrier-Bestandteilen durch die Behandlungskette (12), wobei diese externen Kalibrier-Bestandteile unter den Bestandteilen der Probe ausgewählt werden und eine bekannte Konzentration aufweisen,
- folglich erhalten eines Signals, das für die Konzentrationen der externen Kalibrier-Bestandteile, in Abhängigkeit von mindestens einer Variablen der Behandlungskette (12) und von mindestens einem konstanten Parameter unbekannten Werts und/oder von mindestens einem stabilen statistischen Parameter der Behandlungskette, repräsentativ ist,
- anwenden (204) von mindestens einem Teil des Schätzungs-Schrittes der biologischen oder chemischen Parameter unter Zuhilfenahme der Vorrichtung zur Signalverarbeitung (14) durch Bayessche Inferenz, um daraus den Wert von jedem konstanten Parameter unbekannten Werts und/oder von jedem stabilen statistischen Parameter der Behandlungskette (12) abzuleiten,
- aufzeichnen (206) im Speicher (32) von jedem zuvor abgeleiteten Wert des konstanten Parameters und/oder des stabilen statistischen Parameters.

5. Verfahren zum Schätzen von biologischen oder chemischen Parametern (x, B) nach einem der Ansprüche von 1 bis 4, in dem sich die chemischen und biologischen Parameter (x, B) auf Proteine beziehen und in dem die Probe (E) eines der Elemente aus der Gruppe umfasst, die aus Blut, Plasma und Urin besteht.

6. Verfahren zum Schätzen von biologischen oder chemischen Parametern (x, B) nach einem der Ansprüche von 1 bis 5, in dem:

- das die biologischen oder chemischen Parameter (x, B) darstellende Signal (Y) in Abhängigkeit der molekularen Konzentrationen der Spezies (K) ausgedrückt wird,
- diese Spezies (K) sich aus einem Zerfall der im Fokus stehenden bzw. zentralen molekularen Spezies (x) ergeben,
- das Verfahren eine Schätzung einer Menge diese Spezies umfasst, die sich aus einem Zerfall der zentralen molekularen Spezies (x) ergeben.

7. Verfahren zum Schätzen von biologischen oder chemischen Parametern (x, B) nach Anspruch 6, in dem:

- die Spezies Peptide oder Polypeptide umfassen,
- die zentralen molekulare Spezies Proteine umfassen, die jeweils eine bestimmte Menge an diesen Peptiden oder Polypeptiden enthalten,
- man definiert einen Verdauungsertrag ($\alpha$) der Proteine als Matrix aus Koeffizienten $\alpha_{ip}$, wobei $\alpha_{ip}$ den Verdauungsertrag des p-ten Proteins im Verhältnis zum i-ten Peptid oder Polypeptid darstellt, sodass die molekularen Konzentrationen (K) von Peptiden oder Polypeptiden an einen Vektor (x) gebunden sind, der die Protein-Konzentrationen durch eine Verdauungs-Matrix (D) und diesen Verdauungsertrag ($\alpha$) darstellt,
- das Verfahren eine Schätzung dieses Verdauungsertrags ($\alpha$) umfasst.

8.  Verfahren zum Schätzen von biologischen oder chemischen Parametern (x, B) nach Anspruch 6, in dem:

    - die Spezies Peptide und Polypeptide umfassen,
    - die zentralen molekularen Spezies Proteine umfassen, die eine bestimmte Menge an diesen Peptiden oder Polypeptiden enthalten,
    - man einen Gesamtgewinn ($\xi$) der Behandlungskette (12) definiert, sodass das die biologischen oder chemischen Parameter (x, B) darstellende Signal (Y) durch die Gleichung Y= $\xi$ K dargestellt wird, wobei K ein Vektor ist, der die Konzentrationen der Peptide oder Polypeptide darstellt,
    - das Verfahren eine Schätzung dieses Gesamtgewinns ($\xi$) beinhaltet.

9.  Verfahren zur Unterstützung der Diagnose, das die Schritte eines Verfahrens zur Schätzung von biologischen oder chemischen Parametern (x, B) nach irgendeinem der Ansprüche von 1 bis 8 umfasst, in dem die biologischen oder chemischen Parameter (x, B) der Probe (E) einen biologischen oder chemischen Zustandsparameter (B) mit diskreten Werten umfassen, wobei jeder mögliche diskrete Wert dieses Parameters einem möglichen Zustand der Probe (E) zugeordnet ist, und einen Vektor, der die Konzentrationen der Bestandteile der Probe (E) darstellt, und in dem der die Konzentrationen (x) darstellende Vektor und der biologische oder chemische Zustandsparameter (B) einen probabilistischen Zusammenhang untereinander aufweisen, und wobei die Signalverarbeitung durch Bayessche Inferenz des Weiteren auf der Grundlage einer a priori Wahrscheinlichkeits-Modellierung des die Konzentrationen (x) darstellenden Vektors, bedingt durch die möglichen Werte des biologischen oder chemischen Zustandsparameters (B) realisiert wird.

10. Verfahren zur Unterstützung der Diagnose nach Anspruch 9, das eine vorangehende Lernphase (500) umfasst, die die folgenden Schritte umfasst:

    - aufeinander folgend, eine Vielzahl an Referenz-Proben ($E_{REF}$) durch die Behandlungskette (12) durchlaufen lassen (502), wobei der Wert des biologischen oder chemischen Zustandsparameters (B) einer jeden Referenz-Probe bekannt ist,
    - auf diese Weise ein Signal erhalten, das die Konzentrationen (x) der Bestandteile für jede Referenz-Probe ($E_{REF}$) in Abhängigkeit von mindestens einer Variablen der Behandlungskette (12) darstellt,
    - anwenden (504, 506, 508) von mindestens einem Teil des Schätzungs-Schrittes der biologischen oder chemischen Parameter unter Zuhilfenahme der Vorrichtung zur Signalverarbeitung durch Bayessche Inferenz, um daraus Werte der Konzentrationen der Bestandteile für jede Referenz-Probe ($E_{REF}$) abzuleiten,
    - daraus ableiten (510) von Parametern der a priori WahrscheinlichkeitsGesetzmäßigkeit des die Konzentrationen (x) darstellenden Vektors bedingt durch die möglichen Werte des biologischen oder chemischen Zustandsparameters (B), und
    - aufzeichnen (510) im Speicher (32) dieser Parameter der Wahrscheinlichkeits-Gesetzmäßigkeit.

11. Verfahren zur Unterstützung der Diagnose nach Anspruch 9 oder 10, das eine vorangehende Phase (300) der Auswahl der genannten Bestandteile aus einer Menge aus Bestandteil-Kandidaten umfasst, wobei diese vorangehende Phase (300) der Auswahl die folgenden Schritte umfasst:

    - aufeinander folgend, eine Vielzahl an Referenz-Proben ($E_{REF}$) durch die Behandlungskette (12) durchlaufen lassen (302), wobei der Wert des biologischen oder chemischen Zustandsparameters (B) einer jeden Referenz-Probe bekannt ist,
    - auf diese Weise ein Signal erhalten, das die Konzentrationen (x) der Kandidaten-Bestandteile für jede Referenz-Probe ($E_{REF}$) in Abhängigkeit von mindestens einer Variablen der Behandlungskette (12) darstellt,
    - anwenden (304, 306, 308) von mindestens einem Teil des Schätzungs-Schrittes der biologischen oder chemischen Parameter unter Zuhilfenahme der Vorrichtung zur Signalverarbeitung durch Bayessche Inferenz, um daraus Werte abzuleiten, welche die Konzentrationen (x) der Kandidaten-Bestandteile für jede Referenz-Probe ($E_{REF}$) darstellen,
    - daraus ableiten (310) von Parametern der Verteilungen des Vektors, der die Konzentrationen (x) der Kandidaten-Bestandteile für jeden diskreten Wert des biologischen oder chemischen Zustandsparameters (B) darstellt,
    - auswählen (312, 314), unter den Bestandteil-Kandidaten, diejenigen, deren Verteilungen untereinander die größten Unterschiede aufweisen, in Abhängigkeit der Werte des biologischen oder chemischen Zustandsparameters (B).

12. Vorrichtung (10) zur Schätzung der biologischen oder chemischen Parameter (x, B) in einer Probe (E), das Folgendes umfasst:

- eine Kette (12) zur Behandlung der Probe (E), die zur Bereitstellung eines Signals (Y) konzipiert ist, das die biologischen oder chemischen Parameter (x, B), abhängig von mindestens einer Variablen der Behandlungskette, darstellt,
- eine Vorrichtung zur Signalverarbeitung (14), die, in Kombination mit der Behandlungskette (12), zur Anwendung eines Verfahrens (100) zur Schätzung der biologischen oder chemischen Parameter (x, B) oder zur Unterstützung der Diagnose nach irgendeinem der Ansprüche von 1 bis 11 konzipiert ist.

13. Vorrichtung (10) zur Schätzung der biologischen oder chemischen Parameter (x, B) nach Anspruch 12, bei der die Behandlungskette (12) eine chromatografische Säule (22) und/oder ein Massenspektrometer (26) umfasst, und zur Bereitstellung eines Signals (Y) konzipiert ist, das die Konzentrationen (x) der Bestandteile der Probe (E) in Abhängigkeit einer Retentionszeit (T) in der chromatografischen Säule (22) und/oder des Masse/Ladungs-Verhältnisses im Massenspektrometer (26) darstellt.

**Claims**

1. A method for estimating biological or chemical parameters (x, B) in a sample (E) comprising the following steps:

- put (102) the sample (E) through a processing chain (12),
- obtain a representative signal (Y) of said biological or chemical parameters (x, B) as a function of at least one variable of the processing chain, and
- estimate (104, 106, 108, 110) said biological or chemical parameters (x, B) using a signal processing device (14) by Bayesian inference, on the basis of a direct analytical modeling of said signal (Y) as a function of said biological or chemical parameters (x, B) and as a function of technical parameters ($\gamma_b$, $\xi$, T, K, K*, $\alpha$) of the processing chain (12),

**characterized in that** at least two of said biological or chemical (x , B) or technical ($\gamma_b$, $\xi$, T, K, K*, $\alpha$) parameters as a function of which direct analytical modeling of said signal (Y) is defined have a probabilistic dependence relationship between each other, and **in that** said signal processing by Bayesian inference is furthermore accomplished on the basis of modeling by a conditional prior probability distribution of this dependence.

2. A method for estimating biological or chemical parameters (x, B) according to claim 1, wherein the estimating step (104, 106, 108, 110) of said biological or chemical parameters (x, B) includes, by approximation of the posterior joint probability distribution of said biological or chemical (x, B) and technical ($\gamma b$, $\xi$, T, K, K*, $\alpha$) parameters, conditionally to the obtained signal (Y), using a stochastic sampling algorithm:

- a sampling loop (106) of at least part (x, B) of said biological or chemical parameters of the sample (E) and of at least part ($\gamma_b$, $\xi$, T, K, K*, $\alpha$) of said technical parameters of the processing chain, providing sampled values of these parameters, and
- an estimate (108) of said at least part of said biological or chemical and technical parameters (x, B, $\gamma_b$, $\xi$, T, K, K*, $\alpha$) calculated from said provided sampled values.

3. A method for estimating biological or chemical parameters (x, B) according to claim 2, wherein the estimate (108) of said at least part of said biological or chemical and technical parameters (x, B, $\gamma b$, $\xi$, T, K, $\alpha$) calculated from said provided sampled values comprises:

- a calculation of the expectation or median or maximum a posteriori estimator for each continuous values parameter (x, $\gamma_b$, $\xi$, T, K, K*, $\alpha$),
- a calculation of the maximum a posteriori estimator for each discrete values parameter (B), or
- a probability calculation of at least part of said biological or chemical and technical parameters (x, B, $\gamma_b$, $\xi$, T, K, K*, $\alpha$).

4. A method for estimating biological or chemical parameters (x, B) according to any one of claims 1 to 3, wherein the biological or chemical parameters include a vector representative of concentrations of sample components, said method further including a preliminary calibration phase (200), called external calibration, comprising the following steps:

- put (202) a sample ($E_{CALIB1}$) of external calibration components through the processing chain (12), with these external calibration components chosen from among the components of said sample and whose concentrations are known,
- by this means obtain a signal representative of concentrations of external calibration components as a function of at least one variable of the processing chain (12) and of at least one constant parameter of unknown value and/or of at least one stable statistic parameter of the processing chain,
- apply (204) at least part of said estimating step of said biological or chemical parameters using the signal processing device (14) by Bayesian inference, to infer the value of each constant parameter of unknown value and/or of each stable statistic parameter of the processing chain (12),
- save (206) each constant parameter value and/or each stable statistic parameter value previously inferred in a memory (32).

5. A method for estimating biological or chemical parameters (x, B) according to any of claims 1 to 4, wherein said biological or chemical parameters (x, B) are relative to proteins and the sample (E) includes one of the elements of the group consisting of blood, plasma and urine.

6. A method for estimating biological or chemical parameters (x, B) according to any one of claims 1 to 5, wherein:

- the signal (Y) representative of said biological or chemical parameters (x, B) is expressed as a function of molecular species concentrations (K),
- these species (K) come from a decomposition of molecular species of interest (x),
- the method includes an estimate of the number of said species obtained resulting from said decomposition of molecular species of interest (x).

7. A method for estimating biological or chemical parameters (x, B) according to claim 6, wherein:

- the species contain peptides or polypeptides,
- the molecular species of interest contain proteins that each have a number of these peptides or polypeptides,
- a digestion yield ($\alpha$) of proteins is defined in the form of a coefficients $\alpha_{ip}$ matrix, where $\alpha_{ip}$ designates the digestion yield of the p-th protein with relation to the i-th peptide or polypeptide, such that the molecular concentrations (K) of peptides or polypeptides are linked to a vector (x) representative of protein concentrations via a digestion matrix (D) and said digestion yield ($\alpha$),
- the method includes an estimate of this digestion yield ($\alpha$).

8. A method for estimating biological or chemical parameters (x, B) according to claim 6, wherein:

- the species contain peptides or polypeptides,
- the molecular species of interest contain proteins that each have a number of these peptides or polypeptides,
- an overall gain ($\xi$) of the processing chain (12) is defined so as to model said signal (Y) representative of biological or chemical parameters (x, B) by the relationship $Y = \xi K$, where K is a vector representative of concentrations of peptides or polypeptides,
- the method includes an estimate of this overall gain ($\xi$).

9. A method for aiding diagnosis comprising the steps of a method for estimating biological or chemical parameters (x, B) according to any one of claims 1 to 8, wherein the biological or chemical parameters (x, B) of the sample (E) contain a biological or chemical state parameter (B) with discrete values, with each possible discrete value of that parameter associated with a possible state of the sample (E), and a vector representative of concentrations of components of the sample (E), and wherein since the vector representative of concentrations (x) and the biological or chemical state parameter (B) have a probabilistic dependence among each other, the signal processing by Bayesian inference is furthermore carried out on the basis of modeling by prior probability distribution of the vector representative of concentrations (x) conditionally to possible values of the biological or chemical state parameter (B).

10. A method for aiding diagnosis according to claim 9, including a preliminary learning phase (500) comprising the following steps:

- successively put (502) a plurality of reference samples ($E_{REF}$) through the processing chain (12), with the value of the biological or chemical state parameter (B) known for each reference sample,
- obtain a representative signal of concentrations (x) of the components for each reference sample ($E_{REF}$) depending on at least one variable of the processing chain (12),
- apply (504, 506, 508) at least part of the biological or chemical parameters estimating step using the signal processing device by Bayesian inference to determine values of component concentrations for each reference sample ($E_{REF}$),
- determine (510) parameters of prior probability distribution for the vector representative of concentrations (x) conditionally to possible values of the biological or chemical state parameter (B), and
- save (510) these probability distribution parameters in a memory (32)

11. A method for aiding diagnosis according to claim 9 or 10, including a preliminary phase (300) for selecting said components from a pool of candidate components, said preliminary selection phase (300) including the following steps:

- successively put (302) a plurality of reference samples ($E_{REF}$) through the processing chain (12), with the value of the biological or chemical state parameter (B) known for each reference sample,
- obtain a signal representative of concentrations (x) of the candidate components for each reference sample ($E_{REF}$) as a function of at least one variable of the processing chain (12),
- apply (304, 306, 308) at least part of the biological or chemical parameters estimating step using the signal processing device by Bayesian inference to determine values representative of concentrations (x) of candidate components for each reference sample ($E_{REF}$),
- determine (310) parameters of distribution of the vector representative of concentrations (x) of candidate components for each discrete value of the biological or chemical state parameter (B),
- select (312, 314) from among the candidate components those for which the distributions are the most dissimilar from each other as a function of the biological or chemical state parameter values (B).

12. An estimating device (10) for biological or chemical parameters (x, B) in a sample (E) comprising:

- a processing chain (12) of the sample (E) designed for providing a signal (Y) representative of said biological or chemical parameters (x, B) as a function of at least one variable of the processing chain,
- a signal processing device (14) designed to apply, in combination with the processing chain (12), a method (100) for estimating biological or chemical parameters (x, B) or for aiding diagnosis according to any one of claims 1 to 11.

13. An estimating device (10) for biological or chemical parameters (x, B) according to claim 12, wherein the processing chain (12) includes a chromatography column (22) and/or a mass spectrometer (26) and is designed to provide a signal (Y) representative of concentrations (x) of components of the sample (E) as a function of a retention time (T) in the chromatography column (22) and/or a mass-to-charge ratio in the mass spectrometer (26).

*Figure 1*

*Figure 2*

## Figure 3

# EP 2 509 018 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2028486 A